# EUROPEAN PATENT SPECIFICATION

(11) **EP 3 903 778 B1**
(45) Date of publication and mention of the grant of the patent: **30.07.2025**
(21) Application number: 19901505.8
(22) Date of filing: 30.12.2019
(51) Int. Cl.: A61K 31/35, A61K 31/00, A61K 31/54, A61P 17/00, A61K 31/352, C07D 405/12, C07D 311/92, C07D 407/12, C07D 493/10, C07D 497/10, A61K 9/00, A61K 45/06, A61K 31/5575, A61K 31/365, A61K 31/573, A61P 17/06

(54) **IMMUNOSUPPRESSIVE PHARMACEUTICAL COMPOSITION AND APPLICATION THEREOF**
IMMUNSUPPRESSIVE PHARMAZEUTISCHE ZUSAMMENSETZUNG UND ANWENDUNG DAVON
COMPOSITION PHARMACEUTIQUE IMMUNOSUPPRESSIVE ET SON APPLICATION

(30) Priority: 29.12.2018 CN 201811645299
(43) Date of publication of application: 03.11.2021
(73) Proprietor: Shanghai Archeus Biotech Co., Ltd., Shanghai 201203 (CN)
(72) Inventor: GONG, Xiaoming, Shanghai 201203 (CN)
(74) Representative: Manitz Finsterwald Patent- und Rechtsanwaltspartnerschaft mbB
(86) International application number: PCT/CN2019/129945
(87) International publication number: WO 2020/135872

(56) References cited:
- EP-A2- 0 650 723
- EP-A2- 0 650 723
- WO-A1-2007/123699
- WO-A1-85/03637
- US-A- 4 883 793
- AMMON HERMANN P T ET AL: "REVIEW Forskolin : From an Ayurvedic Remedy to a Modern Agent", PLANTA MEDICA, 1 June 1985 (1985-06-01), pages 473 - 477, XP055946921, Retrieved from the Internet <URL:https://www.thieme-connect.de/products/ejournals/pdf/10.1055/s-2007-969566.pdf> [retrieved on 20220728]
- W. REMY ET AL: "Prostaglandin E2 gel improvement of psoriatic lesions", INTERNATIONAL JOURNAL OF DERMATOLOGY, vol. 25, no. 4, 31 May 1986 (1986-05-31), pages 266 - 268, XP055715063, DOI: 10.1111/j.1365-4362.1986.tb02240.x
- YE, LIUMAO ET AL: "Clinical observation on the action of a compound dexamethasone cream used for treating psoriasis", QINGHAI MEDICAL JOURNAL, vol. 29, no. 8, 20 August 1999 (1999-08-20), CN, pages 12 - 13, XP009521703, ISSN: 1007-3795
- M.B PATEL: "Forskolin: a successful therapeutic phytomolecule", EAST AND CENTRAL AFRICAN JOURNAL OF PHARMACEUTICAL SCIENCES, vol. 13, no. 1, 28 August 2014 (2014-08-28), pages 25 - 32, XP055715071
- YASHASWINI SHARMA ET AL: "Coleus (plectranthus barbatus)–a multipurpose medicinal herb", INTERNATIONAL RESEARCH JOURNAL OF PHARMACY, vol. 2, no. 3, 6 March 2011 (2011-03-06), pages 47 - 58, XP055715073

## Description

### Field of The Invention

The present invention relates to drugs and pharmaceutical compositions for inducing immunosuppression and applications thereof, in particular to drugs, pharmaceutical compositions for use in treating psoriasis or psoriatic arthritis using forskolin derivatives.

### Background of The Invention

Psoriasis is a chronic skin inflammation that seriously affects the quality of human life, and belongs to an autoimmune disease. A common symptom is redness, scaly plaque ringworm on the scalp, elbows, knees, etc., also known as Niupixuan. Histological characteristic of the skin of the lesion are significant thickening of the epidermis, invasion of immune cells in the epidermis, and increased number of epithelial dilated blood vessels, etc.

The incidence of psoriasis in adults is as high as 2 to 4%, and there are as many as 125 million patients worldwide, of which the incidence in Europe and the United States ranks among the top. The global market value of the disease reached 11.3 billion dollars in 2016. Due to the huge unmet medical needs of psoriasis worldwide, its pathogenesis has been a research hotspot in the medical field in the past 30 years. Many studies have shown that the lack of function of the innate immune system and the adaptive immune system leads to the activation of dendritic cells, the imbalance of immune T cells, the secretion of proinflammatory cytokines, the excessive proliferation of keratinocytes and the changes in terminal differentiation, etc., which eventually form subcutaneous inflammation and characteristic plaques of psoriasis. Among them, immune T cells are highly enriched in patients' blood and skin lesions, and further secrete high levels of tumor necrosis factor alpha (TNF-α), interleukin 17A, and the like. Tumor necrosis factor α alone, or in combination with other pro-inflammatory factors such as interleukin 23, interleukin 17, etc., acts to initiate downstream immune responses. Therefore, tumor necrosis factor α, interleukin 17A, and interleukin 23 are all clinicopathological indications and high-value therapeutic targets for psoriasis.

For the target TNF-α, mature products based on macromolecular drugs are already available in the global market, represented by, such as, AbbVie's adalimumab (Humira) and Amgen's Etanercept (Enbrel). The development of small molecule TNF-α inhibitors is challenging in pharmaceutical chemistry. At present, only the phosphodiesterase inhibitor Pentoxifylline is on the market and the anti-inflammatory effect of the secondary development drug Thalidomide is related to the reduction of TNF-α. TNF-α inhibitors are the first class of biological drugs used to treat psoriasis. They have achieved good effects in the treatment of psoriasis in clinical practice by inhibiting the action of TNF-α. For example, TNF-α receptor Fc fusion protein Etanercept has become the standard reference for the development of drugs for psoriasis since its approval in 1998; and TNF-α monoclonal antibody adalimumab has also been upgraded from the second-line drug approval in the clinical system to the first-line drug for moderate to severe chronic plaque psoriasis.

Interleukin 17A is a new target for immune regulation that has gradually received attention since 2000. This is related to an immunological milestone event: the discovery of T helper 17 (Th17 cell) in 2005. With the deepening of research and development in the pharmaceutical industry, from 2015 to 2016, Novartis's Secukinumab and Eli Lilly's Ixekizumab were the first to market as IL-17A monoclonal antibody drugs, and showed superior clinical efficacy and safety in the treatment of psoriasis. Compared with other target drugs, IL-17A antibodies showed a stronger clinical response in moderate to severe psoriasis reaching the psoriasis area and severity index (PASI) PASI90 and PASI100, and the efficacy persisted in both short-term and long-term treatments. Based on the above performance, in 2015, "Nature" magazine evaluated that "anti-interleukin-17 drugs will become the standard treatment regimen for psoriasis". Compared with other targets, interleukin 17A is currently recognized as a psoriasis treatment target with the highest pathological relevance, the most significant clinical response, superior efficacy and high safety.

Nevertheless, regardless of whether it is an interleukin 17A inhibitor or a tumor necrosis factor α inhibitor, the inhibition of a single factor cannot achieve a satisfactory response in 100% of patients with psoriasis, which suggests that the disease involves multiple mechanisms of action and there are other alternative inflammatory signaling pathways. Therefore, recent strategies include the development of other drug forms such as bispecific antibodies to simultaneously inhibit two pro- inflammatory factors. Targeting these two pro-inflammatory factors at the same time is considered to be a strategy that may achieve a better efficacy. There is no marketed product based on this strategy, and it is a high-value market blank area that deserves attention. Only Janssen's COVA322 in research is a bispecific antibody targeting IL-17A and TNF-α, which is currently in the second phase of psoriasis clinical trial and is worth the wait.

It should be emphasized that macromolecular drugs account for the vast majority of drugs on the global market in the above-mentioned field. The reason is that the signal pathways related to pro-inflammatory factors are intricate, and the pharmaceutical industry usually chooses clear and feasible antibody drug development strategies, such as direct binding of pro-inflammatory factors through antibody macromolecules, blocking their binding receptors and then inhibiting downstream functions. However, macromolecular drugs such as antibodies have many shortcomings: in terms of principle and mechanism, macromolecular drugs are easy to have immunogenicity and are difficult to target intracellular targets; in terms of production and application, the preparation process of macromolecular drugs is complicated, the quality standards are difficult to be unified, and transportation costs are high; most importantly, macromolecular drugs are expensive in terms of prices and markets, and are especially not suitable for countries and regions with large populations and heavy medical security burdens such as China; in addition, because oral administration is not possible, they are not suitable for a considerable number of people who can not tolerate drug injections.

Although the potency of small molecule chemical drugs is usually slightly lower than that of antibody drugs, they still have many advantages, such as oral administration, simple preparation process, low production cost, no immunogenicity, and ability to target intracellular targets. Therefore, small molecule drugs that can effectively inhibit TNF-α or/and IL-17A are expected to become a strong competitor or even a market terminator for similar antibody drugs. This field is basically blank in the market at present due to extremely high professional barriers and practical challenges in many processes such as high-through screening, pharmaceutical chemistry, and translational medicine.

In addition to the treatment of psoriasis, immunosuppressive treatment with the basic characteristic and methodology of reducing/inhibiting pro-inflammatory factors is also widely applicable to many other diseases and symptoms, such as inflammation caused by infection or surgical trauma, other autoimmune diseases including rheumatoid arthritis and psoriatic arthritis. Among them, the total market value of various autoimmune diseases based on immunosuppressive therapy exceeds 60 billion dollars. In the case of exogenous pathogen stimulation or increased pathological autoimmune response, lymphoid T cells, macrophages, etc. are activated to secrete pro-inflammatory factors as key signal molecules to initiate the body's inflammatory response, with representative factors being, such as, TNF-α, IL-17, interleukin 6, interferon-γ, etc. Therefore, the increased expression of pro-inflammatory factors is the main pathological indication and cause of the inflammatory response and immune hyperimmune process. Inhibition of pro-inflammatory factors or their receptors through antibody drug binding to achieve immunosuppression is the mainstream strategy in pharmaceutical reaserch and development and in clinical practice. Besides the aforementioned Adalimumab, developed by Abbvie and targeted to inhibit TNF-α, and Secukinuma, developed by Novartis and targeted to IL-17A, for the treatment of rheumatoid arthritis, psoriasis and other autoimmune diseases, there are humanized antibody Reslizumab developed by Teva and targeted to inhibitor of IL-5 for the treatment of asthma, antibody drug Tocilizumab developed by Roche and Genentech and targeted to inhibit IL-6 receptor for the treatment of rheumatoid arthritis, and monoclonal antibody drug Ustekinumab developed by Johnson & Johnson (J&J) and targeted to inhibit IL-12 and IL-23 for the treatment of Crohn's disease, psoriasis, etc.

In addition to the above-mentioned antibody immunosuppressive drugs, there are many small molecule immunosuppressors used to eliminate inflammation (anti-inflammation) and suppress immune function (immunosuppression). Clinically commonly used fast-acting immunosuppressants such as steroids anti-inflammatory drugs Hydrocortisone and methylprednisolone, etc., can inhibit the activity of neutrophils and macrophages by inhibiting cyclooxygenase 2 (COX-2), reduce the pro-inflammatory cytokines TNF-α , IL-1β, etc., and have clinical practice and efficacy in the treatment of rheumatoid arthritis, asthma, Crohn's disease and other autoimmune diseases. In addition, slow-acting immunosuppressants methotrexate, hydroxychloroquine, etc., can inhibit immune cell activity by interfering with the synthesis of immune cell genetic material and excessive cell proliferation, and can also reduce the level of pro-inflammatory factors such as IL-6. They are widely used in the treatment of autoimmune diseases such as rheumatoid arthritis and systemic lupus erythematosus (SLE). However, the above-mentioned small molecule immunosuppressants all have several shortcomings, such as insufficient efficacy, easy to develop drug resistance, and serious side effects of long-term use. The newly developed small molecule drugs for immunosuppression also include JAK (Janus kinase) inhibitors, but they also have the defects of insufficient specificity for different subtypes of JAK and extensive side effects on immune regulation.

EP 0 650 723 A2 discloses the pharmaceutical use of forskolin derivatives or a physiologically acceptable salt thereof as a spasmolytic agent, therapeutic agent for skin ulcer, peripheral circulation improving agent for limbs and differentiation inducing and promoting agent.

In summary, based on the current market characteristics and unmet needs of current global psoriasis drugs and immunosuppressive therapies, small molecule chemical drugs that can effectively inhibit IL-17A or/and TNF-α will become rare species in the market for similar diseases and drugs with the same mechanism. Among them, small molecule compounds, especially those that can effectively inhibit IL-17A and TNF-α at the same time and have dual mechanisms, will have extremely high competitive advantages and subsequent barriers to competition, which will be one of the most promising research and development directions in the field of psoriasis and immunosuppressive therapy.

### Summary of The Invention

The present inventio concerns a pharmaceutical composition for use in treating psoriasis or psoriatic arthritis according to claim 1 and a pharmaceutical composition according to claim 8. Preferred embodiments of the invention are the subject-matter of dependent claims.

### Brief Description of The Drawings

Figure 1 shows the inhibitory effects of different concentrations of 6-[3-(dimethylamino)propionyl]forskolin hydrochloride on the secretion of TNF-α in human monocyte macrophages THP-1 induced by lipopolysaccharide (LPS) . Error bar is STDEV, *: P<0.05, T-test, compared with the group induced by LPS while not treated with 6-[3-(dimethylamino)propionyl]forskolin hydrochloride.
Figure 2 shows the inhibitory effects of different concentrations of forskolin on the secretion of TNF-α in THP-1 cells induced by LPS. Error bar is STDEV, *: P<0.05, T-test, compared with the group induced by LPS while not treated with forskolin. The interval symbol on the abscissa indicates that the 80 nM group was removed from the results due to the amount of LPS being doubled by mistake.
Figure 3 shows the viability level of THP-1 cells treated with 6-[3-(dimethylamino)propionyl]forskolin hydrochloride. Error bar is STDEV.
Figure 4 shows the inhibitory effects of 6-[3-(dimethylamino)propionyl]forskolin hydrochloride and prostaglandin E2 alone and in combination on the secretion of TNF-α in THP-1 cells induced by lipopolysaccharide. Cpd represents compound 6-[3-(dimethylamino)propionyl]forskolin hydrochloride, error bar is STDEV, *: P<0.05, T-test.
Figure 5 shows the viability levels of THP-1 cells treated with 6-[3-(dimethylamino)propionyl]forskolin hydrochloride and prostaglandin E2 alone and in combination. Error bar is STDEV, and Cpd represents compound 6-[3-(dimethylamino)propionyl]forskolin hydrochloride.
Figure 6 shows the inhibitory effects of 6-[3-(dimethylamino)propionyl]forskolin hydrochloride and hydrocortisone alone and in combination on the secretion of TNF-α in THP-1 cells induced by lipopolysaccharide. Cpd represents compound 6-[3-(dimethylamino)propionyl]forskolin hydrochloride, HC represents hydrocortisone, error bar is STDEV, *: P<0.05, T-test.
Figure 7 shows the viability levels of THP-1 cells treated with 6-[3-(dimethylamino)propionyl]forskolin hydrochloride and hydrocortisone alone and in combination. Error bar is STDEV, Cpd represents compound 6-[3-(dimethylamino)propionyl]forskolin hydrochloride, and HC represents hydrocortisone.
Figure 8 shows the inhibitory effects of different concentrations of 6-[3-(dimethylamino)propionyl]forskolin hydrochloride on the secretion of IL-17A in mouse spleen Naïve CD4+ T cells induced to differentiate to Th17 cells by cytokines. Error bar is SEM, *: P<0.05, T-test, compared with the group induced by cytokines while not treated with 6-[3-(dimethylamino)propionyl]forskolin hydrochloride.t
Figure 9 shows that 6-[3-(dimethylamino)propionyl]forskolin hydrochloride has no significant toxic effect on mouse spleen Naïve CD4+ T cells, and the error bar is SEM.
Figure 10 shows the inhibitory effects of 6-[3-(dimethylamino)propionyl]forskolin hydrochloride and prostaglandin E2 alone and in combination on the secretion of IL-17A in mouse spleen Naïve CD4+ T cells induced to differentiate to Th17 cells by cytokines. Cpd represents compound 6-[3-(dimethylamino)propionyl]forskolin hydrochloride, and error bar is SEM, *: P<0.05, T-test.
Figure 11 shows that 6-[3-(dimethylamino)propionyl]forskolin hydrochloride and prostaglandin E2 alone and in combination have no significant toxicity on mouse spleen Naïve CD4+ T cells. Error bar is SEM, and Cpd represents compound 6-[3-(dimethylamino)propionyl]forskolin hydrochloride.
Figure 12 shows the inhibitory effects of 6-[3-(dimethylamino)propionyl]forskolin hydrochloride and hydrocortisone alone and in combination on the secretion of IL-17A in mouse spleen Naïve CD4+ T cells induced to differentiate to Th17 cells by cytokines. Cpd represents compound 6-[3-(dimethylamino)propionyl]forskolin hydrochloride, HC represents hydrocortisone and error bar is SEM, *: P<0.05, T-test.
Figure 13 shows that 6-[3-(dimethylamino)propionyl]forskolin hydrochloride and hydrocortisone alone and in combination have no significant toxicity on mouse spleen Naïve CD4+ T cells. Error bar is SEM, Cpd represents compound 6-[3-(dimethylamino)propionyl]forskolin hydrochloride, and HC represents hydrocortisone.
Figure 14 shows the curve of the skin psoriasis-like index score on the mouse back over time. Error bar is SEM, and significance analysis is shown in Table 10.
Figure 15 shows comparative photographs of the back skins of the mice in each group at the end of 7-day administration.
Figure 16 shows comparative photographs of the high-dose group and the model group on day 3 of the administration. In the photographs, the right ears of the mice are the model sites where imiquimod ointment is applied, and the left ears are the untreated control.
Figure 17 shows the curves of the inflammatory thickening of the skins of the model right ears of the mice. The values are the thickness of the right ears minus the thickness of the left ears, error bar is SEM, and the statistical significance analysis is shown in Table 11.
Figure 18 shows comparative photographs of the right ears of the mice in each group at the end point of the 7-day administration.
Figure 19 shows curves of body weight change of mice in the course of the administration.
Figure 20 shows the results of H&E staining analysis of the skin histology in the model areas on the backs of the mice (x4 objective lens, the displayed are partial images, cropped to the same grid size).

### Detailed Description of The Invention

Unless defined otherwise, all technical and scientific terms used herein have the same meaning as commonly understood by a person of ordinary skill in the art.

Forskolin is a compound extracted from the roots of *Coleus forskohlii* of the Coleus genus in labiatae family in India in the 1970s. It acts as an adenylate cyclase activator and has cardiotonic action, hypotensive effect, etc. Its structural formula is shown in formula II:

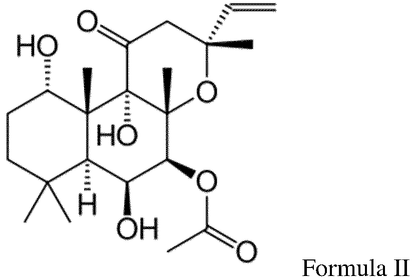

The PCT patent application with application number PCT/US84/00291 reported that a phenomenon was observed in 1985 that forskolin relieved the pathological symptoms of 4 patients with psoriasis. In the same peroid, there were fewer studies showing that forskolin affected the ratio of cAMP to cGMP in epidermal cells by activating cAMP, and further inhibited epidermal cell mitosis in epidermal hyperplasia symptoms such as psoriasis. It should be pointed out that psoriasis is an autoimmune disease, and, in addition to the proliferation of epidermal cells, immune cell invasion and repeated and continuous inflammation at the lesion sites are the most important pathological features. However, the above observations or studies always lacked the explanation and discovery of the mechanism of action (Mechanism of Action) of forskolin on the pathological inflammation of psoriasis. Moreover, because of the extremely limited number of 4 clinical patients, there is no obvious logical connection and sufficient data support. For this reason, the development and application of adenylate cyclase activators represented by Forskolin for the above-mentioned diseases have been stagnant for more than 30 years thereafter. There are many pharmacodynamic studie on Forskolin compound itself, but its poor water solubility limits its further development into medicine. The present invention chooses the structure-optimized adenylate cyclase agonist forskolin structural derivatives, and demonstrates for the first time that forskolin derivatives can specifically reduce the production of pro-inflammatory factor TNF-α in human monocyte macrophage THP-1 *in vitro,* and can specifically reduce the production of pro-inflammatory factor IL-17A during the induced differentiation of Naïve CD4+ T cells from mouse spleen to Th17. The above two inhibitory effects are synergistically enhanced when combined with prostaglandin E2 or hydrocortisone. At the same time, the present invention also demonstrates for the first time that the forskolin derivatives have an efficacy equivalent to that of the first-line clinical drug calcipotriol betamethasone in the imiquimod mouse psoriasis model, and have fewer side effects.

Based on a series of *in vitro* and *in vivo* pharmacodynamic evaluation studies, the present disclosure reveals for the first time that structural derivatives of the small molecule compound forskolin can effectively reduce the secretion level of tumor necrosis factor α in THP-1 human macrophages in pharmacodynamic experiments *in vitro.* When used alone, forskolin structure derivatives can reduce the basic level secretion of TNF-α by up to 75%, and show dose-effect dependence (Concentration response). When combined with prostaglandin E2 or hydrocortisone, they can further reduce the basic level secretion of TNF-α by 90%, showing a stronger pharmacodynamic activity. At the same time, the structural derivatives of the small molecule compound forskolin can effectively reduce the secretion level of interleukin 17A in the *in vitro* pharmacodynamic experiment of inducing the differentiation of Naïve CD4+ T cells from mouse spleen to Th17. When the forskolin derivatives are used alone, they can reduce the basic level secretion of IL-17A by up to 97%, and there is dose-effect dependence (Concentration response). When combined with prostaglandin E2 or hydrocortisone, they can further reduce the basic level secretion of IL-17A, showing astronger pharmacodynamic activity. Furthermore, in a mouse psoriasis model induced by Imiquimod, the structural derivatives of forskolin show the effect of slowing down a number of disease severity indexes. For example, psoriasis-like skin indiction score, inflammatory thickening of the ears of the model, histological change of inflamed skin of the model were changed correspondingly in high and low dose administration groups and combined administration groups. Among them, the efficacy in the groups administrated with PGE2 is comparable to the current first-line clinical external drug calcipotriol betamethasone, while inflammatory redness of the skin is weaker, and there is no side effect of calcipotriol betamethasone, such as, reduction of the body weights of mice. The above findings suggest that the structural derivatives of forskolin, when used alone or in combination, are expected to become a new class of drugs for the treatment of psoriasis and for immunosuppression, and have comparable efficacy and fewer side effects compared with current clinical first-line drugs.

The forskolin derivatives in accordance with the present invention are 6-(4-aminobutyryl)forskolin, 6-[4-(dimethylamino)butyryl]forskolin, 6-[3-aminopropionyl]forskolin, 6-[3-(methylamino)propionyl]forskolin, and 6-[3-(dimethylamino)propionyl]forskolin; their structural formulae are shown in Table 1.

It should also be pointed out that HIL568 is also a forskline derivative later developed by Hoechest for the treatment of glaucoma. It can be speculated that as an adenylate cyclase activator, it also has the effect of inhibiting TNF-α and IL-17A and it therefore has a certain value in the treatment field of the present invention. However, because there is no follow-up development report, it is speculated that the compound has defects such as druggability.

**Table 1. Structures of preferred forskolin derivatives**

| Compound names | structures | data of cAMP activation effect | source |
|---|---|---|---|
| 6-(4-aminobutyryl)forskol in | 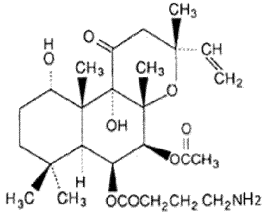 | myocardial contraction activity: 0.9 dp/dt | [1] |
| | | antihypertensive effect: 0.7 | |
| | | cAMP activation 214.9% | |
| 6-[4-(dimethylamino)buty ryl]forskolin | 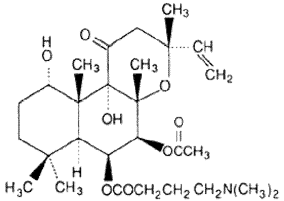 | myocardial contraction activity: equivalent to 100% of forskolin | [2] |
| | | antihypertensive effect: equivalent to 90% of forskolin | |
| 6-[3-(dimethylamino)prop ionyl]forskolin | 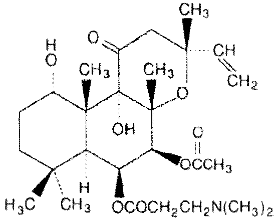 | myocardial contraction activity: equivalent to 120% of forskolin | [2] |
| | | ntihypertensive effect: equivalent to 80% of forskolin | |
| 6-[3-(methylamino)propio nyl]forskolin | 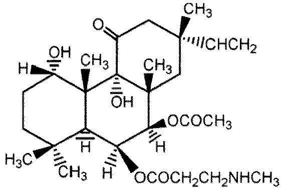 | *in vivo* active metabolite of 6-[3-(dimethylamino)propio nyl]forskolin, equivalent to 6-[3-(dimethylamino)propio nyl]forskolin in beagle dog's systolic blood pressure (SBP), diastolic blood pressure (DBP), heart rate (HR) and other effects | [3] |
| 6-[3-aminopropionyl]fors kolin | 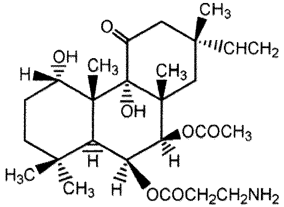 | *in vivo* active metabolite of 6-[3-(dimethylamino)propio nyl]forskolin | [4] |
| 6-[(piperidino)acetyl]-7-desacetyl forskolin (not part of the invention) | 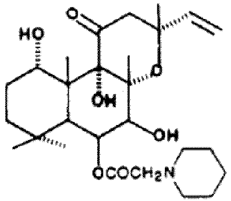 | guinea pig myocardial contraction activity EC50: 1.8 µg/mL | [5] |
| 6-[(1-piperidino)propionyl forskolin (not part of the invention) | 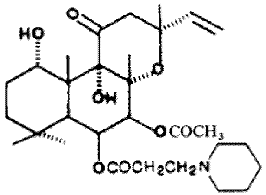 | adenylate cyclase activation EC50= 1-2 µM | [6] |
| | | increase of cyclic guanylic acid EC50= 3µM | |
| 7-(2-formyloxy)forskolin (not part of the invention) | 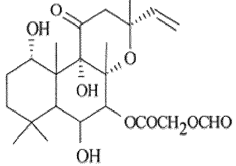 | myocardial contraction activity EC50=0.002 µg/mL | [7] |
| forskolin (not part of the invention) | 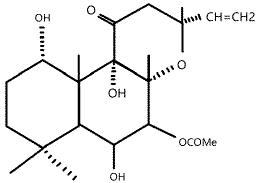 | activation of adenylate cyclase in rat cerebral cortex EC50=8 µM | [8] |
| | | myocardial contraction activity EC50=0.02 µg/mL | |
| 7-(2-methyl)forskolin (not part of the invention) | 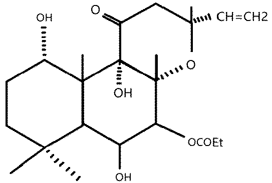 | activation of adenylate cyclase in rat cerebral cortex EC50=15 µM | [8] |
| 7-(2-oxoethyl)forskolin (not part of the invention) | 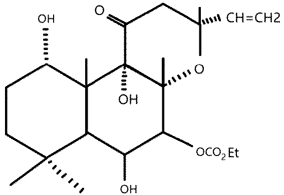 | activation of adenylate cyclase in rat cerebral cortex EC50=8 µM | [8] |

In a particularly preferred embodiment, 6-[3-(dimethylamino)propionyl]forskolin hydrochloride (CAS number: 138605-00-2) of the following formula III is used:

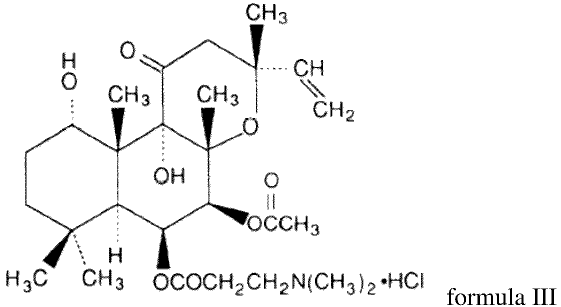

The present invention concerns a pharmaceutical composition including a forskolin derivative, selected from the group consisting of 6-(4-aminobutyryl)forskolin, 6-[4-(dimethylamino)butyryl]forskolin, 6-[3-aminopropionyl]forskolin, 6-[3-(methylamino)propionyl]forskolin, and 6-[3-(dimethylamino)propionyl]forskolin, or a pharmaceutically acceptable salt thereof; and a prostaglandin compound or a glucocorticoid compound.

In some embodiments, the present disclosure also considers a drug or a pharmaceutical composition comprising a prodrug of a forskolin derivative, which are the esters or amides of the forskolin derivative, and a prostaglandin compound or a glucocorticoid compound.

These drugs or pharmaceutical compositions can be used for inducing immunosuppression in a subject in need thereof.

"Pharmaceutically acceptable salts" herein refer to inorganic or organic acid addition salts that are basically harmless to animals or humans, such as hydrochloride, hydrobromide, nitrate, perchlorate, phosphate, sulfate, formate, acetate, aconate, ascorbate, benzenesulfonate, benzoate, cinnamate, citrate, enanthate, fumarate, glutamate, hydroxyacetate, lactate, maleate, malonate, mandelate, methanesulfonate, naphthalene-2-sulfonate, phthalate, salicylate, sorbate, stearate, succinate, tartrate, p-toluenesulfonate, etc. In some embodiments, it is particularly advantageous that the salts of the forskolin derivatives are their hydrochloride. Such salts can be formed by methods well known to those skilled in the art.

The term "co-administration", for example, for a pharmaceutical combination of a forskolin derivative and a prostaglandin compound, includes the forskolin derivative and the prostaglandin compound being administered sequentially and separately, for example, the prostaglandin compound is administered before or after the administration of the forskolin derivative; it also includes the simultaneous administration of the forskolin derivative and the prostaglandin compound in the same pharmaceutical preparation or in the form of separate pharmaceutical preparations. In the embodiment of sequential administration, usually the forskolin derivative and the prostaglandin compound coexist in the subject at least part of the time. In some embodiments of co-administration, the forskolin derivative and the prostaglandin compound may have a synergistic effect, for example, the amount of one compound is lower than the therapeutically effective amount when administered alone, or preferably, amounts of the two compounds are all lower than the therapeutically effective amounts when used alone.

In some embodiments, the present disclosure provides a pharmaceutical kit comprising a forskolin derivative, selected from the group consisting of 6-(4-aminobutyryl)forskolin, 6-[4-(dimethylamino)butyryl]forskolin, 6-[3-aminopropionyl]forskolin, 6-[3-(methylamino)propionyl]forskolin, and 6-[3-(dimethylamino)propionyl]forskolin, or a pharmaceutically acceptable salt thereof, such as hydrochloride, and a prostaglandin compound or a glucocorticoid compound. The pharmaceutical kit can be used for inducing immunosuppression in a subject.

In some embodiments of the pharmaceutical kit, the forskolin derivative or a pharmaceutically acceptable salt thereof and the prostaglandin compound or the glucocorticoid compound can be formulated in a pharmaceutical composition, for example, they are mixed and coexist in the same dosage form or unit dosage form. In other embodiments of the pharmaceutical kit, the forskolin derivative or its pharmaceutically acceptable salt and the prostaglandin compound or glucocorticoid compound can be separately formulated and stored. For example, the forskolin derivative or its pharmaceutically acceptable salt and the prostaglandin compound are both in the form of a solution in different containers, or the forskolin derivative or its pharmaceutically acceptable salt is formulated as an injection, and the prostaglandin compound is formulated as an ointment.

In some embodiments, the pharmaceutical kit provided by the present disclosure may include at least two separate kits, one of which includes the forskolin derivative or a pharmaceutically acceptable salt thereof, and the other includes the prostaglandin compound or glucocorticoid compound. The pharmaceutical kit may also include instructions for simultaneously or sequentially administering the two kits to the subject.

In the drugs, pharmaceutical compositions or pharmaceutical kits provided in the present disclosure, the forskolin derivative or a pharmaceutically acceptable salt thereof, as well as the prostaglandin compound and the glucocorticoid compound may be formulated with a pharmaceutically acceptable carrier. "Pharmaceutically acceptable carrier" herein refers to solid or liquid diluents, fillers, antioxidants, stabilizers and other substances that can be safely administered to animals or humans without excessive adverse side effects, and at the same time, it is suitable for maintaining the activity of the drugs or active agents therein. Depending on the route of administration, various carriers well known in the art can be used, including, but not limited to, sugars, starch, cellulose and its derivatives, maltose, gelatin, talc, calcium sulfate, vegetable oils (such as castor oil), synthetic oil, polyol, alginic acid, phosphate buffer, emulsifier, isotonic saline, and/or pyrogen-free water, etc. Suitable administration routes include, for example, oral, intravenous infusion, intramuscular injection, subcutaneous injection, subperitoneal, rectal, sublingual, or inhalation, transdermal, and other routes. Correspondingly, the forskolin derivative or a pharmaceutically acceptable salt thereof, as well as the prostaglandin compound and the glucocorticoid compound can be formulated together with these pharmaceutically acceptable carriers into any clinically acceptable dosage form, such as tablets, granules, powders, capsules, injection preparations, suppositories, drops, external plasters, ointments, medicated oils, or sprays, etc.

As used herein, "subject" refers to an individual (preferably human) who has or is suspected of suffering from a certain disease (in accordance with the invention psoriasis or psoriatic arthritis), or, for example, when predicting the risk of a disease, the "subject" may also include healthy individuals. This term can often be used interchangeably with "patient", "test subject", "treatment subject" and so on. As used herein, "therapeutically effective amount" refers to an amount sufficient to cause a biological or medical response expected by a clinician in the body of the subject, and it can usually be determined by those skilled in the art according to the route of administration, the weight, age, condition of the subject and other factors. For example, a typical daily dose may range from 0.01 mg to 100 mg of active ingredient per kg body weight. The present disclosure also considers other dosages.

In some specific embodiments, the present disclosure provides a drug comprising 6-[3-(dimethylamino)propionyl]forskolin hydrochloride itself or a pharmaceutical composition together with prostaglandin E2 or hydrocortisone, as well as methods or uses thereof for inducing immunosuppression in a subject.

In some embodiments, the present disclosure provides an immunosuppressive agent comprising a forskolin derivative, selected from the group consisting of 6-(4-aminobutyryl)forskolin, 6-[4-(dimethylamino)butyryl]forskolin, 6-[3-aminopropionyl]forskolin, 6-[3-(methylamino)propionyl]forskolin, and 6-[3-(dimethylamino)propionyl]forskolin, or a pharmaceutically acceptable salt thereof, and optionally a prostaglandin compound or a glucocorticoid compound. In some embodiments, the immunosuppressive agent is a TNF-α inhibitor. In some embodiments, the immunosuppressive agent is an IL-17A inhibitor. In some embodiments, the immunosuppressant suppresses the expression or secretion of TNF-α and IL-17A at the same time.

In some embodiments, the prostaglandin compound is selected from the group consisiting of prostaglandin E2 (PGE2), dinoprost tromethamine, carboprost, carboprost tromethamine, prostaglandin E1 (Alprostadil), bimatoprost, iloprost, limaprost, limaprost α cyclodextrin (Limaprostalfadex), misoprostol, gemeprost, latanoprost, sulprostone, ornoprostil and pharmaceutically acceptable salts thereof.

In some embodiments, the glucocorticoid compound is selected from the group consisiting of dexamethasone, hydrocortisone, prednisone, prednisolone, paramethasone, cortisone, betamethasone, meprednisone, fludrocortisone, triamcinolone acetonide and pharmaceutically acceptable salts thereof.

In some embodiments of the pharmaceutical composition for use of the present disclosure, the forskolin derivative or a pharmaceutically acceptable salt thereof acts by inhibiting the expression or secretion of TNF-α by immune cells, especially monocyte macrophages. In some embodiments thereof, the forskolin derivative or a pharmaceutically acceptable salt thereof acts by inhibiting the secretion of IL-17A by immune cells, especially T lymphocytes. In some embodiments thereof, the forskolin derivative or a pharmaceutically acceptable salt thereof acts by inhibiting the expression or secretion of TNF-α and IL-17A by immune cells.

"Immunesuppression" as used herein refers to the reduction of undesirable immune responses in a subject, including the production of some cytokines such as TNF-α or IL-17A. In some embodiments, the subject in need of immunosuppression is a patient with an autoimmune disease. In some embodiments, the subject in need of immunosuppression is a patient with psoriasis. In other embodiments, the subject in need of immunosuppression is a patient with inflammation.

It should be understood that all drugs that have an anti-inflammatory effect by reducing the body's own immune response rather than inhibiting the activity of foreign pathogens (such as antibiotics) are essentially immunosuppressive drugs. In this case, the concept of anti-inflammatory drugs is equivalent to immunosuppressants.

In summary, the forskolin derivatives involved in the present disclosure can have immunosuppressive functions by inhibiting the production of pro-inflammatory cytokines TNF-α and/or IL-17A, and have potential value in treating or alleviating inflammation and various autoimmune diseases. Based on a new molecular target and mechanism, the forskolin derivatives are expected to become a new type of immunosuppressant to make up for the shortage of current drugs.

The present invention reveals for the first time that structural derivatives of the small molecule compound forskolin have a dual-action mechanism of simultaneously reducing interleukin 17A and tumor necrosis factor α, and has multiple immunosuppressive effects compared with current single-action mechanism drugs on the market. At the same time, the compound itself has been fully optimized in structure and modified in properties, and possesses considerable druggability. It is also a rare small molecule chemical drug species in the drug market with similar action mechanisms and for related diseases. In summary, forskolin structural derivatives have great potential to effectively fill the gaps in the existing market in the field of psoriasis and immunosuppressive therapy.

The present invention is further illustrated with the following examplest.

### Examples

### Example 1 In vitro cytology experiment: Regulatory effect of 6-[3-(dimethylamino)propionyl]forskolin hydrochloride on TNF-α

### 1.1 Experimental materials and main equipments are shown in Table 2 below.

**Table 2. Materials and equipments used in the in vitro cytology experiment**

| materials | brands | catalog No. |
|---|---|---|
| RPMI1640 medium | Thermo | 11835030 |
| Fetal Bovine Serum | Thermo | 10099133 |
| phosphate buffer (DPBS) | Thermo | 14190144 |
| Double antibiotics | Thermo | 15070063 |
| human monocyte macrophage (THP-1) | BeNa Culture | BNCC337680 |
| lipopolysaccharide (LPS) | Sigma | L6529-1MG |
| phorbol ester (PMA) | Sigma | P8139-1MG |
| human TNF-α ELISA kit | Abcam | ab181421 |
| cell viability assay kit (MTT) | Beyotime | C0009 |
| prostaglandin E2 (PGE2) | MCE | HY-101952 |
| Forskolin | Sigma | F6886 |
| NKH 477 | Sigma | N3290 |
| Hydrocortisone | MCE | HY-N0583 |
| microplate reader | TECAN | infinite M1000 |
| cell incubator | Thermo | Forma 371 |
| ultra-clean worktable | BIOBASE | BBS-V800 |
| invert microscope | Novel | NIB-100 |
| centrifuge | Sigma | 2-5 |
| 96-well culture plate | Costar | T3603 |
| T75 culture flask | Corning | 430641 |

### 1.2 Experimental steps

### 1.2.1 Cell culture, drug treatment and cell viability assay:

1) Recoveried and expanded THP-1 cells in a T75 culture flask. The growth medium was 10 milliliters of 10% fetal bovine serum/RPMI1640 (already containing 2 mM glutamine)/1% double antibiotics in each flask. Observed the cells under an invert microscope, and, when the cells were in the logarithmic growth phase, started the following drug treatment experiments;
2) Cultured 2*10e5/mL THP-1 cells in a 96-well culture plate with 100 microliters of growth medium (10% fetal bovine serum/RPMI1640/1% double antibiotics) per well, and added 100 ng/ml PMA to incubate for 24 hour;
3) Eluted the non-adherent cells once with 300 microliters of fresh growth medium of 10% fetal bovine serum/RPMI1640/1% double antibiotics;
4) Added 200 microliters of growth medium of 10% fetal bovine serum/RPMI1640/1% double antibiotics, with/without test compound, to pre-incubate the cells at 37°C for 2 hours;
5) Then added 100 ng/mL lipopolysaccharide (LPS) and incubated at 37°C for 4 hours;
6) Collected 50 microliters of cell culture supernatant for the detection of the levels of related cytokines by enzyme-linked immunoassay, or stored it in a refrigerator at minus 80 degrees Celsius for future detection;
7) Added 10 microliters of 5 mg/ml MTT solution to each cell, and incubated for 4 hours in the cell incubator;
8) Added 100 microliters of Formazan dissolving solution to the cells of each well, and continued to incubate in the cell incubator for about 4 hours, until it was observed under a common optical microscope that Formazan crystals are completely dissolved;
9) Detected the absorbance at 570 nm with a microplate reader.

### 1.2.2 Detecting cytokine expression levels by enzyme-linked immunoassay:

1) Prepared 1X elution buffer for enzyme-linked immunoassay. Prepared it with ultrapure water: 10X elution buffer mother solution = 9:1 in volume ratio for use;
2) Prepared the antibody cocktail for enzyme-linked immunoassay. Prepared it with the antibody diluent (provided by Ab221825): antibody 1: antibody 2 = 18: 1: 1 in volume ratio for use. Antibody 1 was a TNF-α capture antibody and Antibody 2 was a TNF-α detector antibody. They were all supplied by kit Ab221825;
3) Prepared a series of concentration gradient samples of TNF-α standard with ultrapure water for the plotting of reaction standard curve and determining the linear range of detection signal;
4) Added 50 microliters of cell culture supernatant/standard and 50 microliters of the above antibody cocktail to a 96-well plate used for enzymatic reaction, sealed it with a film, placed it on a horizontal shaker, and incubated at 400 rpm for 1 hour at room temperature to allow the antibody-antigen complex was fully bound and coupled at the bottom of the well plate;
5) Discarded the supernatant, eluted with 350 microliters of 1X elution buffer/well for 3 times. In the last elution, put the well plate upside down on an absorbent paper and sucked and washed it thoroughly;
6) Added 100 microliters of TMB substrate solution (supplied by Ab221825) to each well, placed them on a horizontal shaker and incubated in the dark at room temperature for 5 minutes at a speed of 400 rpm;
7) Added 100 microliters of reaction stop solution (provided by Ab221825), placed on a horizontal shaker and mix at 400 rpm for 1 minute;
8) Detected the absorbance at 450 nm with a microplate reader.

### 1.3 Experimental results

### 1.3.1 6-[3-(dimethylamino) propionyl] forskolin hydrochloride reduced the expression of TNF-α in monocyte macrophages

The compound 6-[3-(dimethylamino) propionyl] forskolin hydrochloride (Colforsin daropate hydrochdeloride) was able to concentration-dependently reduce the expression level of TNF-α in human monocyte macrophage THP-1 cultured *in vitro* after stimulation by lipopolysaccharide (LPS), and the reduction of TNF-α is not due to the compound's influence on the cell viability of THP-1.

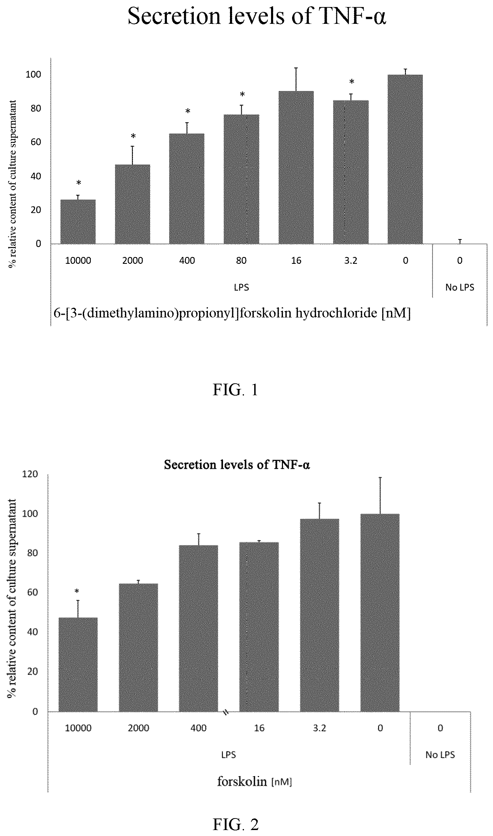

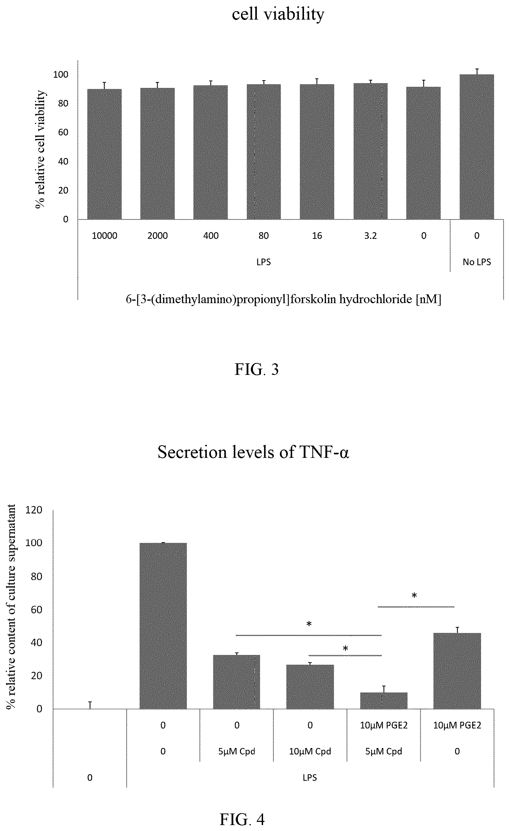

As shown in Figure 1, THP-1 cells cultured *in vitro* showed a significantly increased TNF-α level in the culture supernatant after stimulation with 100 ng/ml LPS for 4 hours, while the compound 6-[3-(dimethylamino) propionyl] forskolin hydrochloride could reduced LPS-induced TNF-α secretion in a concentration-dependent manner in the concentration range of 10 µM to 3 nM. At the highest concentration of 10 µM, it could inhibit about 75% of the TNF-α level of the positive control (the TNF-α level induced by LPS without drug treatment). We also investigated forskolin, a compound of the same structural series, which similarly showed the effect of reducing TNF-α (Figure 2), but the drug activity was slightly lower than the aforementioned compound. In order to further investigate whether this effect of TNF-α reduction was due to the dose-dependent decrease of the viability of THP-1 cells casused by the compound 6-[3-(dimethylamino) propionyl] forskolin hydrochloride, at the end point of the experiment of stimulating with LPS and representative compound 6-[3-(dimethylamino) propionyl] forskolin hydrochloride for 4 hours, we added 10 microliters of 5mg/mL MTT solution to the cell culture supernatant to detect the cell viability level. It was finally determined that there was no significant difference in cell viability between the groups with or without the compound (Figure 3), and thus confirmed that the effect of 6-[3-(dimethylamino) propionyl] forskolin hydrochloride in reducing TNF-α secretion occured through specific immune regulation pathways rather than indirectly caused by impaired cell viability.

### 1.3.2 6-[3-(dimethylamino) propionyl] forskolin hydrochloride combined with prostaglandin E2 (PGE2) reduced the expression of TNF-α in monocyte macrophages

When prostaglandin E2 (PGE2) was used in combination with 6-[3-(dimethylamino) propionyl] forskolin hydrochloride, it could strengthen the effect of 6[3-(dimethylamino) propionyl] forskolin hydrochloride in reducing the level of TNF-α.

As shown in Figure 4, we applied 5 µM and 10 µM compound 6-[3-(dimethylamino) propionyl] forskolin hydrochloride to THP-1 cells cultured *in vitro* and caused the elevated TNF-α level indced by 100ng/mL lipopolysaccharide to drop to 33% and 27% of the positive control respectively.In order to investigate whether the combined use of this compound and prostaglandin E2 could enhance the above-mentioned effect of the compound, we used 10 µM PGE2 and 5 µM 6-[3-(dimethylamino) propionyl] forskolin hydrochloride in combination, and the results showed that the addition of 10 µM PGE2 allowed an inhibition of TNF-α level from a 67% reduction when 6-[3-(dimethylamino) propionyl] forskolin hydrochloride used alone to to a 90% reduction in TNF-α level. The effect of the combined use was better than the inhibitory effect of the higher concentration of 10 µM 6-[3-(dimethylamino) propionyl] forskolin hydrochloride (73%), showing the advantage of the combined use over increased dose. At the same time, we also detected the effect of adding 10 µM PGE2 alone, and found that PGE2 also inhibited the secretion and expression of TNF-α to a certain extent. We speculated that this was related to the fact that it similarly increased the intracellular cAMP level. However, compared with the same concentration of 6-[3-(dimethylamino) propionyl] forskolin hydrochloride, PGE2 alone did not show a stronger inhibitory effect, which suggested that PGE2 alone lacked advantages. Moreover, it ruled out the possibility that the enhanced effect of 6-[3-(dimethylamino) propionyl] forskolin hydrochloride in combination with PGE2 in inhibiting TNF-α was unilaterally dominated by PGE2 itself.

In order to further clarify that the combined effect of 6-[3-(dimethylamino) propionyl] forskolin hydrochloride and PGE2 was a synergistic effect rather than a purely additive effect, according to the judgment principle for synergistic effect of compounds in reference [9-11]: **expected value of combined treatment with A and B** = (value of treatment with A alone /control group value)* (value of treatment with B alone/control group value) *control group value, and the ratio of **expected value of combined treatment with A and B/ ratio of actual value of combined treatment with A and B** is the **Combination Index.** If the Combination Index is greater than 1, then the compounds A and B have a synergistic effect, otherwise, it is a purely additive effect. According to this principle and the data in Table 3 below, it was obtained through calculation that the combined effect of 6-[3-(dimethylamino)propionyl]forskolin hydrochloride and PGE2 was a synergistic effect.

**Talbe 3. Results of the combined use 6-[3-(dimethylamino)propionyl]forskolin hydrochloride and PGE2**

| TNF-α | A (not part of the invention) | B (not part of the invention) | A+B in combination (part of the invention) | combination idex |
|---|---|---|---|---|
| Treatment group | 6-[3-(dimethylamino)propiony l]forskolin hydrochloride | prostaglandin E2 (PGE2) | 6-[3-(dimethylamino)propionyl] forskolin hydrochloride + PGE2 | A * B / (A+B in combination) |
| treatment value | 33% | 46% | 10% | 1.52 |

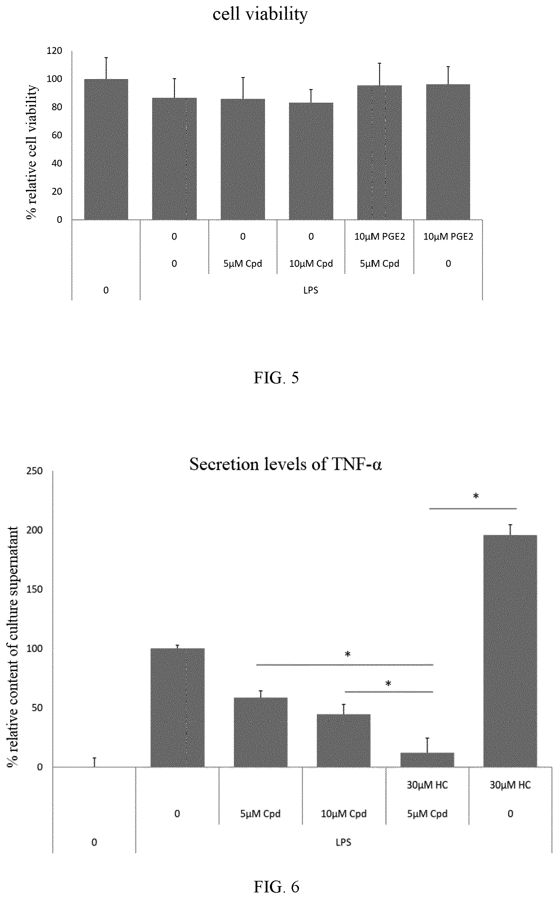

Similarly, we added the same amount of MTT solution as mentioned before at the end point of the drug stimulation in the above experiment. The data showed that cell viability was not affected by the drug treatment, and there was no significant difference between the groups (Figure 5), thus confirming that the regulation of TNF-α expression was the result of the regulation in related signal pathways by the above-mentioned drugs, and not resulted indirectly from impaired cell viability.

### 1.3.3 6-[3-(dimethylamino)propionyl]forskolin hydrochloride combined with hydrocortisone (HC) reduced the expression of TNF-α in monocyte macrophages

When hydrocortisone was used in combination with 6-[3-(dimethylamino)propionyl]forskolin hydrochloride, it could strengthen the compound's effect in reducing TNF-α.

Hydrocortisone is a glucocorticoid drug commonly used clinically to treat psoriasis and for immunosuppression. We also investigated whether it had a synergistic effect with forskolin (or its derivatives) pharmacodynamically. As shown in Figure 6, we applied 5 µM and 10 µM of compound 6-[3-(dimethylamino)propionyl]forskolin hydrochloride to THP-1 cells cultured *in vitro,* and inhibited the elevated TNF-α indced by 100 ng/mL LPS to 58% and 44% of the positive control level, respectively. In order to investigate whether hydrocortisone could enhance the effect of this compound, we used 30 µM hydrocortisone and 5 µM of this compound in combination, and the results showed that the addition of 30 µM hydrocortisone allowed a further inhibitory effect of 6-[3-(dimethylamino)propionyl]forskolin hydrochloride from a 42% reduction with 5 µM alone to a 88% reduction with the combination in TNF-α levels. The effect of the combination was better than the inhibitory effect of a higher concentration of 10 µM 6-[3-(dimethylamino)propionyl]forskolin hydrochloride alone (56%), showing that the combined use of both achieved a better effect than that resulted from increasing the dose of a single compound.At the same time, we also detected the effect of 30 µM hydrocortisone alone. Interestingly, hydrocortisone is an anti-inflammatory glucocorticoid drug commonly used in clinical practice; however, in this experimental system it had an opposite effect that it further stimulated the increase of inflammatory factor TNF-α. It was previously reported that hydrocortisone might have a biphasic effect when the relative order of administration time and inflammation occurrence time was different. In our experimental system for induction of TNF-α factor expression *in vitro,* a "preventive" drug interference was utilized, in which hydrocortisone or a test compound was pre-incubated with cells for 2 hours prior to the inflammation stimulus lipopolysaccharide. After the subsequent addion of the inflammation stimulus lipopolysaccharide, it might be that hydrocortisone promoted a higher level of TNF-α factor expression at this time point, and achieved the effect of enhancing the occurrence of inflammatory response to completely resist the invasion of inflammatory stimulus LPS, which exactly suggested that there might be a mechanism defect in the long-term repeated use of hydrocortisone in the treatment of chronic inflammation such as psoriasis. Nevertheless, based on the above experimental data, we could conclude that hydrocortisone combined with 6-[3-(dimethylamino)propionyl]forskolin hydrochloride could enhance the effect of reducing the TNF-α level by 6-[3-(dimethylamino)propionyl]forskolin hydrochloride, and this enhancement was not dominated by hydrocortisone itself. Similarly, through calculation and analysis in Table 4 below, we determined that the combined effect of 6-[3-(dimethylamino)propionyl]forskolin hydrochloride and hydrocortisone was a synergistic effect.

**Table 4. Results of the combined use of 6-[3-(dimethylamino)propionyl]forskolin hydrochloride and hydrocortisone**

| TNF-α | A | B | A+B in combination | combination idex |
|---|---|---|---|---|
| Treatment group | 6-[3-(dimethylamino)propi onyl]forskolin hydrochloride (not part of the invention) | hydrocortison e (HC) (not part of the invention) | 6-[3-(dimethylamino)propionyl]forsk olin hydrochloride + hydrocortisone (part of the invention) | A*B/ (A+B in combination) |
| treatment value | 58% | 200% | 12% | 9.67 |

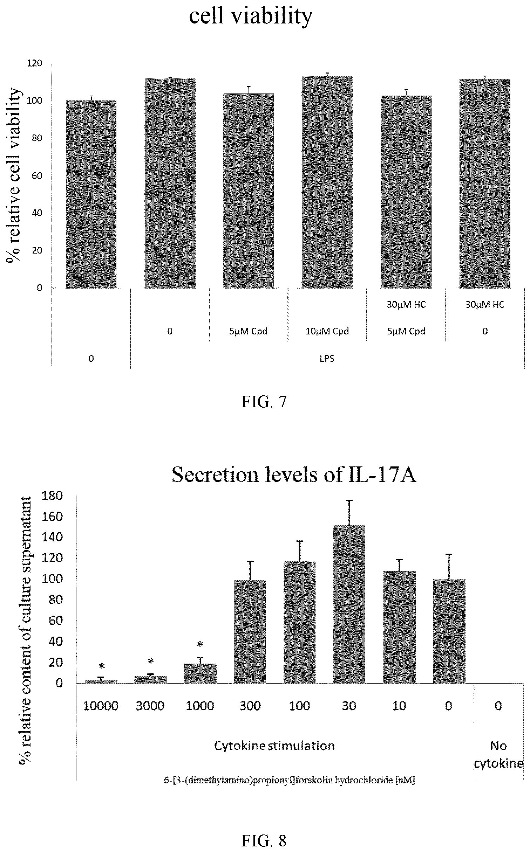

We also added the same amount of MTT solution as mention before at the end point of the drug stimulation in the above-mentioned experiment. The data results ruled out the possibility that cell viability was affected by drug treatment (Figure 7), confirming that the changes in TNF-α expression resulted from the regulation of immune regulation-related signal pathways by the above drugs.

### Example 2 In vitro cytology experiment: Regulatory effect of 6-[3-(dimethylamino)propionyl]forskolin hydrochloride on IL-17A

### 2.1 The experimental materials and main equipments are shown in Table 5 below_{∘}

**Table 5. Materials and equipments used in the in vitro cytology experiment**

| materials | brands | catalog Nos. |
|---|---|---|
| RPMI 1640 medium | Gibco | 11875-093 |
| FBS | Biological Industries | 04-002-1A |
| phosphate buffer (DPBS) | Biosera | LM-S2041/500 |
| double antibiotics | Gibco | 15140122 |
| mTGF-β | R&D | 7666-MB |
| mIL-6 | Peprotech | 216-16 |
| mIL-23 | R&D | 1887-ML |
| mTNF-α | Peprotech | 315-01A |
| mIL-1β | Peprotech | 211-11B |
| anti-mouse IL-4 | Biolegend | 504102 |
| anti-mouse IFN-r | Biolegend | 505702 |
| anti-mouse CD3e | BD Biosciences | 553057 |
| anti-mouse CD28 | BD Biosciences | 553294 |
| Stimulation cocktail | eBioscience | 00-4970-93 |
| RoboSep^{™} Buffer | Stemcell | 20104 |
| Mouse Naïve CD4+ T Cell Isolation Kit | STEMCELL | 19765A |
| prostaglandin E2 (PGE2) | MCE | HY-101952 |
| NKH 477 | Sigma | N3290 |
| Hydrocortisone | MCE | HY-N0583 |
| 96-well flat-bottom cell culture plate | Corning | 3599 |
| Mouse IL-17 DuoSet ELISA | R&D | DY421 |
| CytoTox-one Homogeneous Membrane Integrity Assay Kit | Promega | G7891 |

Animal strain: adult C57BL/6 female mice, 8-12 weeks old.

### 2.2 Experimental steps

### 2.2.1 Isolation and culture of mouse primary T cells, Th17 cell differentiation and compound treatment

1) Pre-coated the 96-well plate with 2µg/mL anti-mouse CD3e and incubated overnight at 4 degrees Celsius;
2) Separated fresh spleens from adult C57BL/6 female mice (8-12 weeks old), ground and filtered through a 70 µM nylon cell strainer to obtain a single cell suspension in pre-cooled DPBS;
3) Washed the spleen cells again with DPBS, and resuspended in RoboSepTM buffer at a density of 1x10⁸/mL;
4) Separated primary CD4+ T cells with mouse primary CD4+ T cell isolation kit as required by the kit;
5) Resuspended the isolated CD4+ T cells in complete medium (RPMI 1640 medium + 10% inactivated FBS + 1% double antibiotics) at a density of 1x10⁶/mL;
6) Washed the 96-well plate pre-coated with anti-mouse CD3e twice with DPBS, added 50 µL of cytokine cocktail, the composition and final concentration of which are shown in Table 6 below, and then added 50 µL of compound solution (the final concentration of DMSO was 0.1%). Added 100 µL of cell suspension to each well to allow the final cell number per well to be 1 x 10⁵/100 µL;

**Table 6 Composition of the cytokine cocktail**

| composition | mTGF-β | mIL-6 | Anti-mIL-4 | Anti-IFN-γ | mIL-23 | mTNF-α | mIL-1β | Anti-mCD3e (Pre-coat) | Anti-mCD28 (Soluble) |
|---|---|---|---|---|---|---|---|---|---|
| final concentration | 5 ng/mL | 50 ng/mL | 10 µg/mL | 10 µg/mL | 10 ng/mL | 10 ng/mL | 10 ng/mL | 2 µg/mL | 2 µg/mL |

7) Cultured the cells for 7 days under the above conditions. The culture conditions were 37 degrees Celsius, 5% CO₂ concentration;
8) After 7 days, added 1X stimulation cocktail (eBioscience 500X) to the culture medium for 4 hours, and then collected cell culture supernatant for detection with IL-17A enzyme-linked immunoassay kit;
9) Completed the detection of supernatant IL-17A concentration with mouse IL-17 DuoSet ELISA kit;
10) Read the OD450nm absorbance value with a microplate reader, and generated the standard curve by using the 4-parameter logistic fitting (4-PL) method.

### 2.2.2 Detecting expression levels of cytokine IL-17A by enzyme-linked immunoassay

1) Diluted the capture antibody (provided in the kit) to a working concentration in PBS without carrier protein, that is, a 96-well plate can be coated with 100 microliters per well. Sealed and incubated the plate overnight at room temperature;
2) Aspirated the coating buffer of the well plate, and eluted with 400 microliters of eluent (provided in the kit) for three times, aspirating as much liquid as possible each time;
3) Added 300 microliters of diluent (provided in the kit) to each well, and equilibrated for 1 hour at room temperature;
4) Eluted the well plate as in the above step 2) to be ready for the addition of sample;
5) Added 100 microliters of sample or standard to each well containing the added diluent, and sealed and incubated the well plate for 2 hours at room temperature;
6) Eluted the well plate as in the above step 2);
7) Added 100 microliters of detector antibody (provided in the kit) to each well, and sealed and incubated the well plate for 2 hours at room temperature.
8) Eluted the well plate as in the above step 2);
9) Added 100 microliters of Streptavidin-HRP working solution (provided in the kit) to each well, and sealed and incubated the well plate in dark for 20 minutes at room temperature.
10) Eluted the well plate as in the above step 2);
11) Added 100 microliters of reaction substrate solution (provided in the kit) to each well, and incubated at room temperature for 20 minutes, protected from light;
12) Added 50 microliters of stop solution (provided in the kit) to each well, and tapped the well plate to ensure that the liquid is evenly mixed;
13) Detected the optical density value of each well at 450 nm. The values could be corrected by deducting the values at 540nm or 570nm.

### 2.2.3 Compound cytotoxicity detection

1) Separated fresh spleens from adult C57BL/6 female mice (8-12 weeks old), and ground and filtered through a 70 µM nylon cell strainer to obtain a single cell suspension in pre-cooled PBS;
2) Seeded primary CD4+ T cells in 96-well plate with a density of 1X10⁵/90µL. The medium was 1640 medium (serum-free);
3) Added the serially diluted compounds formulated in a volume of 10 µL to designated wells;
4) Incubated the cells for 4 hours at 37 degrees Celsius and 5% CO₂;
5) Detected lactate dehydrogenase (LDH) release according to CytoTox-one Homogeneous Membrane Integrity Assay Kit instructions.

### 2.3 Experimental results

### 2.3.1 6-[3-(dimethylamino)propionyl]forskolin hydrochloride reduced the expression of IL-17A in mouse Th17 cells induced to differentiate

Compound 6-[3-(dimethylamino)propionyl]forskolin hydrochloride (Colforsin daropate hydrochdeloride) could concentration-dependently reduce the secretion and expression of IL-17A in mouse primary CD4+ T cells induced to differentiate under the combined action of cytokines TGF-β, IL-6, IL-23, etc., and the reduction in IL-17A was not due to the toxicity of the compound to Th17 cells.

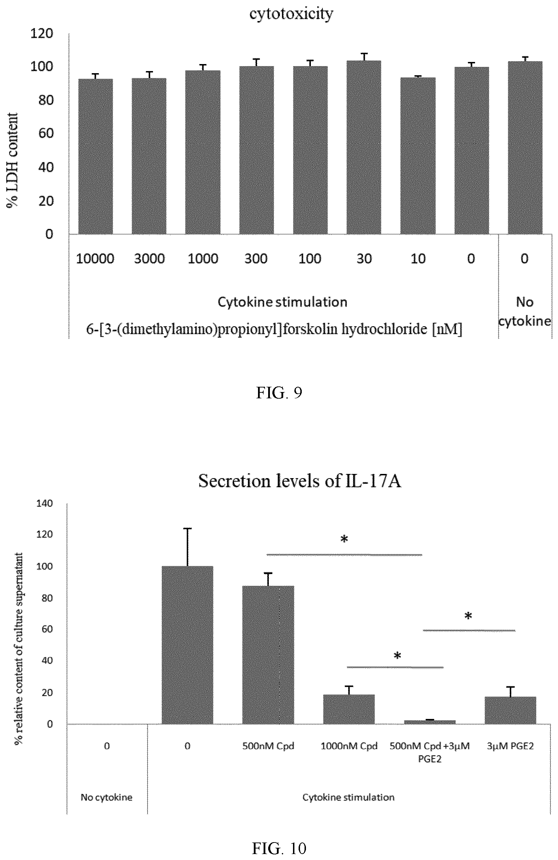

As shown in Figure 8, mouse primary CD4+ T cells showed a significantly increased level of IL-17A in the culture supernatant under the induction of cytokine combinations for 7 days, while the compound 6-[3-(dimethylamino)propionyl]forskolin hydrochloride concentration-dependently reduced the induced basic IL-17A secretion in the concentration range of 10 µM to 10 nM, and could inhibit about 97% of the positive control IL-17A level (the level of IL-17A induced by the cytokine combination without drug treatment) at the highest concentration of 10 µM. In order to further investigate whether such effect of IL-17A reduction was caused by the toxicity of the compound 6-[3-(dimethylamino)propionyl]forskolin hydrochloride weakening the viability of CD4+ T cells, we separately tested the toxicity of the compound on CD4+ T cells. The compound was diluted in the culture medium and incubated with CD4+ T cells for 4 hours, and the concentration of lactate dehydrogenase released into the culture medium was measured. The results showed that the compound was not significantly toxic to the cells (Figure 9), thus confirming that the effect of 6-[3-(dimethylamino) propionyl] forskolin hydrochloride in reducing IL-17A secretion occured through specific immune regulation pathways rather than indirectly caused by impaired cell viability.

### 2.3.2 6-[3-(dimethylamino)propionyl]forskolin hydrochloride combined with prostaglandin E2 (PGE2) reduced the expression of IL-17A in mouse Th17 cells induced to differentiate

When prostaglandin E2 (PGE2) was used in combination with 6-[3-(dimethylamino)propionyl]forskolin hydrochloride, it could strengthen the action of 6[3-(dimethylamino)propionyl]forskolin hydrochloride in reducing IL-17A levels.

As shown in Figure 10, during the induced differentiation to Th17 from mouse primary CD4+ T cells, we applied 0.5 µM and 1 µM of the compound 6-[3-(dimethylamino)propionyl]forskolin hydrochloride and resulted in the elevated IL-17A levels induced by cytokine combination to drop to 88% and 19% of the positive control (no compound treatment). In order to investigate whether a combined use of this compound and prostaglandin E2 could enhance the above-mentioned effects of the compound, we used 3 µM PGE2 and 0.5 µM 6-[3-(dimethylamino)propionyl]forskolin hydrochloride in combination. The results showed that the addition of 3 µM PGE2 allowed 0.5 µM 6-[3-(dimethylamino)propionyl]forskolin hydrochloride to further inhibit IL-17A levels from a 12% reduction when used alone to a 97% reduction in IL-17A levels. Moreover, the effect of the combined use was better than the inhibitory effect of the higher concentration of 1 µM 6-[3-(dimethylamino)propionyl]forskolin hydrochloride alone (81%), showing an advantage of the combined use over increased dose alone. At the same time, we also tested the effect of adding 3 µM PGE2 alone, and found that PGE2 also inhibited the secretion and expression of IL-17A to a certain extent. We speculated that this was related to the fact that it similarly increased the intracellular cAMP level. However, the inhibitory effect of 3 µM PGE2 was comparable to the lower concentration of 1 µM 6-[3-(dimethylamino)propionyl]forskolin hydrochloride (inhibit to be 17% and 18% of the basic level, respectively). It showed that PGE2 alone did not have a stronger inhibitory effect, suggesting that PGE2 alone lacked advantages, and it further ruled out the possibility that the enhanced effect of 6-[3-(dimethylamino) propionyl] forskolin hydrochloride in combination with PGE2 in inhibiting IL-17A was unilaterally dominated by PGE2 itself.

Similarly, in order to further clarify that the combined effect of 6-[3-(dimethylamino)propionyl]forskolin hydrochloride and PGE2 in inhibiting the secretion of IL-17A was a synergistic effect rather than a purely additive effect, we similarly calculated the relevant combination index as shown in the table below. According to the foregoing principles and the data in Table 7 below, it was obtained through calculation that the combined effect of 6-[3-(dimethylamino)propionyl]forskolin hydrochloride and PGE2 in reducing IL-17A was a synergistic effect.

**Table 7. Results of the combined use 6-[3-(dimethylamino)propionyl]forskolin hydrochloride and PGE2**

| IL-17A | A | B | A+B in combination | combination idex |
|---|---|---|---|---|
| Treatment group | 6-[3-(dimethylamino)propio nyl]forskolin | prostaglandin E2 (PGE2) (not | 6-[3-(dimethylamino)propionyl] forskolin hydrochloride + | A * B / (A+B in combination) |
| | hydrochloride (not part of the invention) | part of the invention) | PGE2 (part of the invention) | |
| treatment value | 88% | 17% | 2% | 7.48 |

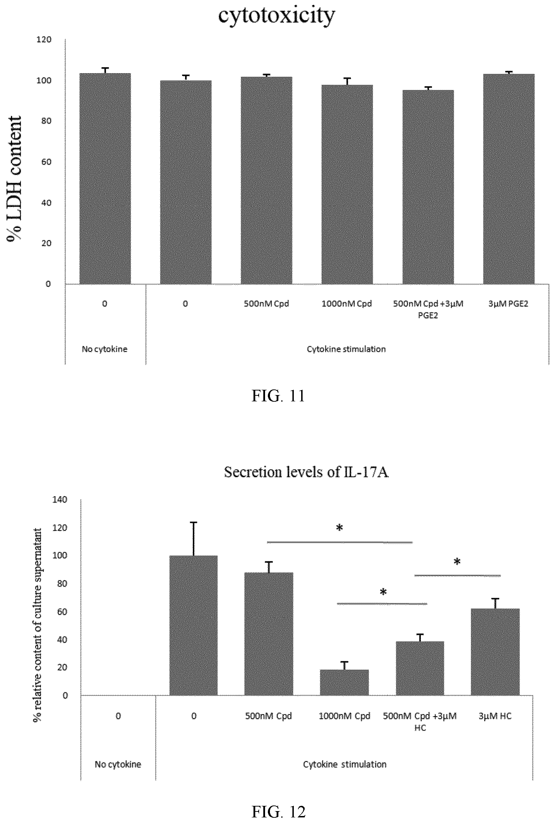

Similarly, we tested the toxic effect of the compounds on mouse CD4+ T cells under the aforementioned experimental dosing conditions. The compounds were incubated with cells for 4 hours to detect the concentration of lactate dehydrogenase released in the supernatant. The data showed that cell viability was not affected by drug treatment and there was no significant difference between groups (Figure 11), thus comfirming that the regulation of IL-17A expression was the result of the regulation in related signal pathways by the above-mentioned drugs, and not resulted indirectly from impaired cell viability.

### 2.3.3 6-[3-(dimethylamino)propionyl]forskolin hydrochloride combined with hydrocortisone (HC) reduced the expression of IL-17A in mouse Th17 cells induced to differentiate

When hydrocortisone was used in combination with 6-[3-(dimethylamino)propionyl]forskolin hydrochloride, it could strengthen the action of 6[3-(dimethylamino)propionyl]forskolin hydrochloride in reducing IL-17A levels.

Hydrocortisone is a glucocorticoid drug commonly used clinically to treat psoriasis and for immunosuppression. We also investigated whether it had a synergistic effect with forskolin derivatives pharmacodynamically. As shown in Figure 12, during the induced differentiation to Th17 from mouse primary CD4+ T cells, we applied 0.5 µM and 1 µM of the compound 6-[3-(dimethylamino)propionyl]forskolin hydrochloride and resulted in the elevated IL-17A levels induced by cytokine combination to drop to 88% and 19% of the positive control (no compound treatment group). In order to investigate whether hydrocortisone could enhance the effects of this compound, we used 3 µM hydrocortisone and 0.5 µM of this compound in combination. The results showed that the addition of 3 µM hydrocortisone allowed a further inhibitory effect of 0.5 µM 6-[3-(dimethylamino)propionyl]forskolin hydrochloride on IL-17A from a 12% reduction with 0.5 µM alone to a 61% reduction with the combination in IL-17A levels. At the same time, we also tested the effect of 3 µM hydrocortisone alone and it reduced the IL-17A level by 38% of the basic level.

Similarly, we calculated and analyzed in Table 8 below to clarify that the combined effect of 6-[3-(dimethylamino)propionyl]forskolin hydrochloride and hydrocortisone in reducing IL-17A secretion was a synergistic effect.

**Table 8. Results of the combined use 6-[3-(dimethylamino)propionyl]forskolin hydrochloride and hydrocortisone**

| IL17-A | A | B | A+B in combination | combination idex |
|---|---|---|---|---|
| Treatment group | (dimethylamino)propi onyl]forskolin hydrochloride (not part of the invention) | hydrocortisone (HC) (not part of the invention) | (dimethylamino)propionyl] forskolin hydrochloride + hydrocortisone (part of the invention) | A * B / (A+B in combination) |
| treatment value | 88% | 62% | 39% | 1.40 |

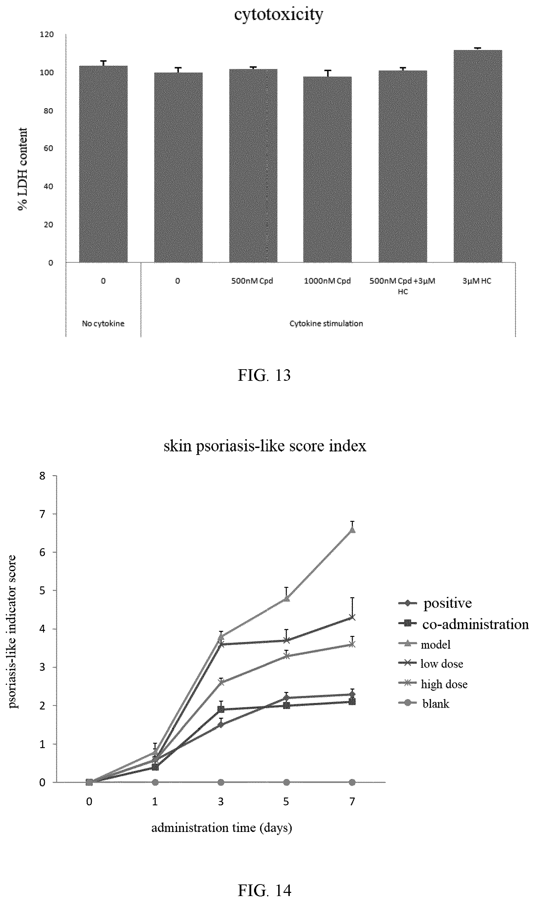

Similarly, we tested the toxic effect of the compounds on mouse CD4+ T cells under the aforementioned experimental dosing conditions. The compounds were incubated with cells for 4 hours to detect the concentration of lactate dehydrogenase released in the supernatant. The data showed that cell viability was not affected by drug treatment and there was no significant difference between groups (Figure 13), thus comfirming that the regulation of IL-17A expression was the result of the regulation in related signal pathways by the above-mentioned drugs, and not resulted indirectly from impaired cell viability.

### Example 3 In vivo disease model experiment

### 3.1 The experimental materials and main equipments are shown in Table 9.

**Table 9. Materials and equipments used in the in vivo disease model experiment.**

| materials | brands | catalog Nos. (or drug registration Nos.) |
|---|---|---|
| 5% imiquimod cream | Aldara | H20160079 |
| calcipotriol betamethasone ointment | LEO | H20160204 |
| phosphate buffer (DPBS) | Thermo | 14190144 |
| prostaglandin E2 (PGE2) | MCE | HY-101952 |
| 75% ethanol | Greagent | G73537N |
| 4% paraformaldehyde fixative | Beyotime | P0099-100ML |
| digital display micrometer thickness gauge | SYNTEK | CLXL005 |
| electric mouse hair scraper | Zhongke life | 3303 |

Animal strain: 28 male BALB/c mice, 6-8 weeks old, SPF environment, 12 hours light and dark cycle, 24-26 degrees Celsius.

### 3.2 Experiment groups:

Blank group: no treatment, n=3;
Positive drug group: calcipotriol betamethasone ointment, n=5;
Model group: 5% imiquimod ointment, n=5;
High-dose compound test group: 3.5mg/kg drug intraperitoneal injection + 5% imiquimod ointment, n=5;
Low-dose compound test group: 0.8mg/kg drug intraperitoneal injection + 5% imiquimod ointment, n=5;
Combined drugs test group: 0.8mg/kg drug intraperitoneal injection + 0.0025% PGE2 (administered epidermally in 75% ethanol) + 5% imiquimod ointment, n=5;

### 3.3 Experimental steps:

### Blank group:

Day 0: anesthetized the mouse, shaved the back with an area about 2*3 cm;
Day1-7: every other day, measured the mouse body weight, left and right ear thickness, and observed the skin to obtain a score;
Day7: killed the mouse, sampled the back skin and fixed in 4% PFA solution for H&E staining;

### Model group induced with imiquimod:

Day 0: anesthetized the mouse, shaved the back with an area about 2*3 cm;
Day1-7: every other day, measured the mouse body weight, left and right ear thickness, and observed the skin to obtain a score;
Day1-7: at a fixed time each day, applied 100 µL and 10 µL of 75% ethanol on the back and right ear on model sites respectively, and massaged briefly until the liquid evaporated off;
Day1-7: then injected 200 µl sterile DPBS intraperitoneally as a drug solvent control;
Day1-7: then applied 62.5 mg of imiquimod ointment on the shaved area on the back of each mouse, applied 250 µg of imiquimod ointment to the inside and outside of the ear on the right ear, and massaged against the direction of hair to help absorption;
Day7: killed the mouse, sampled the back skin and fixed in 4% PFA solution for H&E staining;

### Positive drug group:

Day 0: anesthetized the mouse, shaved the back with an area about 2*3 cm;
Day1-7: every other day, measured the mouse body weight, left and right ear thickness, and observed the skin to obtain a score;
Day1-7: At a fixed time each day, applied 18 mg calcipotriol betamethasone ointment on the shaved area on the back of each mouse, and 1.8 mg calcipotriol betamethasone ointment on the inside and outside of the right ear. Massaged moderately against the direction of the hair to help absorption;
Day 1-7: After calcipotriol betamethasone ointment was administered for 1 hour and the complete absorption was observed by naked eyes, applied 62.5 mg imiquimod ointment on the shaved area on the back of each mouse, and 250 µg imiquimod on the inside and outside of the right ear. Massaged moderately against the direction of the hair to help absorption;
Day7: killed the mouse, sampled the back skin and fixed in 4% PFA solution for H&E staining;

### High-dose compound group:

Day 0: anesthetized the mouse, shaved the back with an area about 2*3 cm;
Day1-7: every other day, measured the mouse body weight, left and right ear thickness, and observed the skin to obtain a score;
Day1-7: at a fixed time each day, applied 100 µL and 10 µL of 75% ethanol on the back and right ear on model sites respectively, and massaged briefly until the liquid evaporated off;
Day1-7: then injected intraperitoneally 3.5 mg/kg in 200 µL sterile DPBS;
Day1-7: then applied 62.5 mg of imiquimod ointment on the shaved area on the back of each mouse, applied 250 µg of imiquimod ointment on the inside and outside of the right ear, and massaged moderately against the direction of hair to help absorption;
Day7: killed the mouse, sampled the back skin and fixed in 4% PFA solution for H&E staining;

### Low-dose compound group:

Day 0: anesthetized the mouse, shaved the back with an area about 2*3 cm;
Day1-7: every other day, measured the mouse body weight, left and right ear thickness, and observed the skin to obtain a score;
Day1-7: at a fixed time each day, applied 100µL and 10µL of 75% ethanol on the back and right ear on model sites respectively, and massaged briefly until the liquid evaporates off;
Day1-7: then injected intraperitoneally 0.8 mg/kg in 200µL sterile DPBS;
Day1-7:then applied 62.5 mg of imiquimod ointment on the shaved area on the back of each mouse, applied 250 µg of imiquimod ointment on the inside and outside of the right ear, and massaged moderately against the direction of hair to help absorption;
Day7: killed the mouse, sampled the back skin and fixed in 4% PFA solution for H&E staining;

### Group treated with compound in combination with PGE2:

Day 0: anesthetized the mouse, shaved the back with an area about 2*3 cm;
Day1-7: every other day, measured the mouse body weight, left and right ear thickness, and observed the skin to obtain a score;
Day1-7: At a fixed time each day, applied 100 µL and 10 µL of 0.0025% PGE2 (2.5 µg on the back of each mouse, in 75% ethanol) on the back and ear model sites respectively, and massaged briefly until the liquid evaporates off;
Day1-7: then injected intraperitoneally 0.8 mg/kg in 200 µL sterile DPBS;
Day 1-7: one hour after PGE2 administration, applied 62.5 mg of imiquimod ointment on the shaved area on the back of each mouse, and applied 250 µg of imiquimod ointment on the inside and outside of the right ear. Massaged moderately against the direction of hair to help absorption;
Day7: killed the mouse, sampled the back skin and fixed in 4% PFA solution for H&E staining;

### 3.4 Experimental results

The experimental indicator used was the psoriasis-like index score:
Four aspects of lesion skin area, plaque, redness and rash. The area was fixed in the disease model and was not considered, and the other three aspects were quantified as follows: 0 = asymptomatic, 1 = mild, 2 = moderate, 3 = significant, 4 = very significant;
Changes of the thickening of the ear;
Body weight;
Skin histological changes (H&E staining).

In the experiment of mouse psoriasis model induced with imiquimod, we used the groups with 6-[3-(dimethylamino)propionyl]forskolin hydrochloride at a high dose of 3.5 mg/kg and a low dose of 0.8 mg/kg (not part of the invention), and at the same time designed a combined administration group of 0.8 mg/kg 6-[3-(dimethylamino)propionyl]forskolin hydrochloride and 0.0025% prostaglandin E2 (PGE2). The positive treatment group was the current first-line clinical topical combination medicine calcipotriol betamethasone ointment. During 7 consecutive days of administration, we observed that the groups with the high and low doses of 6-[3-(dimethylamino)propionyl]forskolin hydrochloride and the combined administration group with PGE2 could all show an effect of slow down various psoriasis-like indicators in a certain period of time. Moreover, compared with the positive control first-line medicine, the combined administration group had better effects as to indicators such as avoiding mouse weight loss, skin inflammation and redness.

As shown in Figure 14, the psoriasis-like index of the skin of the model area on the back of the mouse gradually increased with the increase of the administration time. The statistical significance analysis was shown in Table 10.

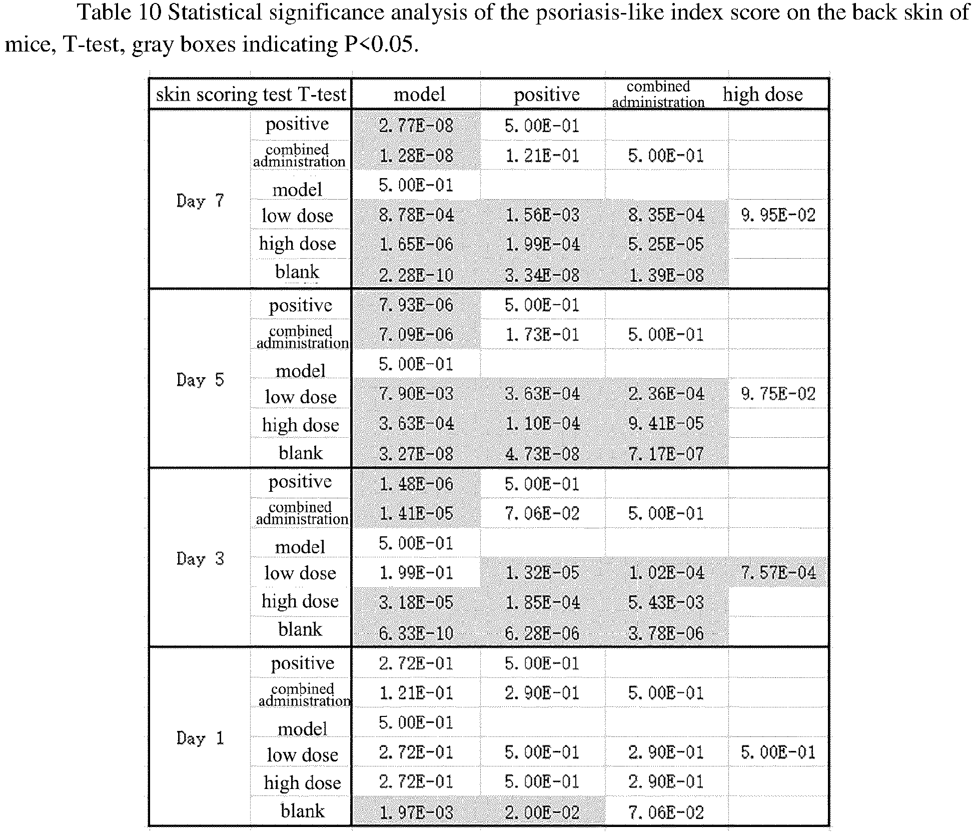

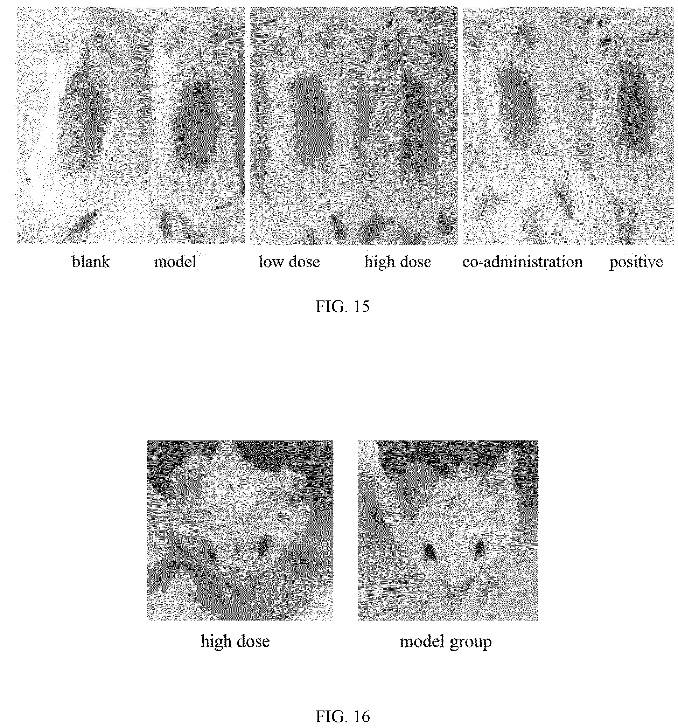

The model group was manifested by the severity of skin scurf, redness and rash (Figure 15). The group with the high-dose 6-[3-(dimethylamino)propionyl]forskolin hydrochloride exhibited a faster effect than that of the low-dose group, and showed an effect of disease alleviation on day 3 of the administration, while the low-dose group began to exhibite the effect on day 5. However, the difference in efficacy between the high-dose group and the low-dose group tended to shrink at the end point of day 7, suggesting that although the high-dose group exhibited a fast effect, the efficacies of high and low doses might be the same under long-term administration. In addition, the effect in the combined administration group was better than that in the high-dose group, which was consistent with the *in vitro* experimental results in the Examples, and its improved effect was equivalent to that of the positive drug calcipotriol betamethasone ointment. It could be seen from Figure 15 that compared to the model group induced by imiquimod and groups with high- or low-dose alone, the back skin of the mouse in the combined administration group was smooth and free of plaques, and the skin redness was lighter than that of the positive control group. Table 10 showed the statistical differences of back psoriasis-like scores between the groups. Combined with Figure 14, we could conclude that the groups with high or low dose of 6-[3-(dimethylamino)propionyl]forskolin hydrochloride alone (not part of the invention) and the combined administration group could all significantly slow down the development of psoriasis-like pathological characteristics of the mouse back model skin, and the combined administration group had the same effect as the current first-line drug calcipotriol betamethasone.

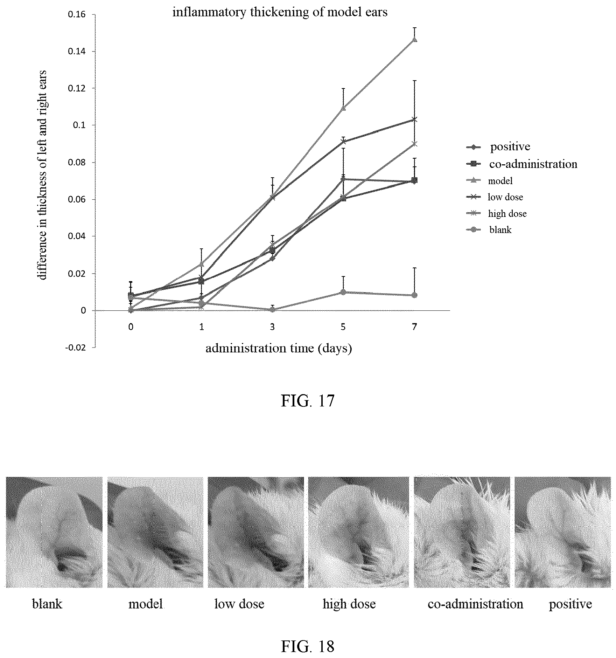

In addition, as shown in Figure 16 and Figure 17, we measured the thickness of the left and right ears of the mice in parallel, and the results showed that, compared with untreated left ears, the model right ears induced by imiquimod exhibited characteristics such as inflammatory thickening, vasodilatation and skin redness. The characteristics were gradually enhanced during the 7-day continuous period of imiquimod administration. Both the high-dose and low-dose groups of the drug and the combined administration group could all show the effects of relieving the inflammation, thickening and redness of the right ear induced by imiquimod within a certain period of time. At the end point of 7-day administration, the combined administration had the strongest effect (relieving by 52%), similar to the positive drug (relieving by 53%). The high-dose group (relieving by 39%) and the low-dose group (relieving by 30%) had slightly weaker effects, but there was no significant difference between the high and low dose groups, suggesting that the difference in the effects of high and low doses might be reduced in the later stage of continuous administration, similar to the experimental results of the back scores. Table 11 listed the significant difference analysis values between the groups in Figure 17.

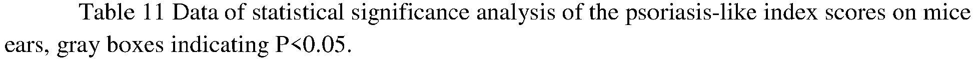

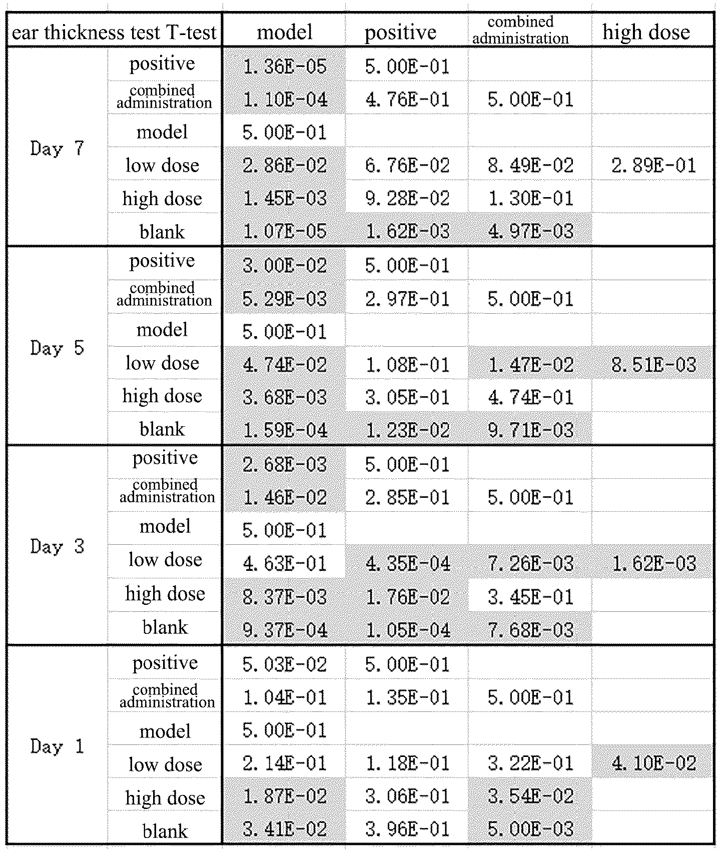

Figure 18 showed different degrees of skin inflammation and redness of the model right ears of each treatment group at the end point of 7-day administration.Figure 16 showed the comparison of redness on the left and right ears of the same mouse on day 3 of the experiment, suggesting that the high-dose group could exhibit the relief effect earlier on day 3 of modeling.

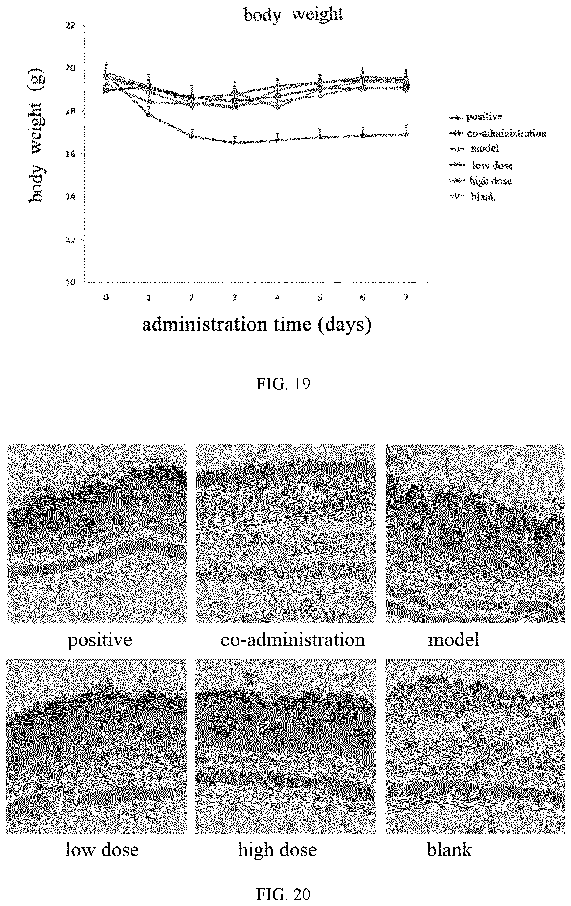

We also analyzed the body weight changes of the mice during the administration period (Figure 19). Except for the positive control group, in which calcipotriol betamethasone treatment resulted in weight loss of the mice, the mice in the other groups showed no obvious changes in body weight. This was consistent with existing research reports and related pre-experimental results of this research system. The hormone components in the positive combination drug would reduce the weight of mice, and it was positively related to its dosage. This suggested that the current first-line medication calcipotriol betamethasone still had its side effects of long-term clinical use, while the ingredients and combination of the present invention did not show an effect on body weight. It was expected to overcome the side effects of existing first-line drugs and became a better medication choice.

The histological analysis of the skin of the back model area (Figure 20) showed that compared with the blank group, the epidermis and stratum corneum of the mouse back skin were thickened in the model group, while there were different degrees of thickness reduction in the groups with high and low doses alone, the combined administration group and the positive control group.

The above exampls are only used to illustrate the technical solutions of the present invention, which is defined by the appended claims.

### Refernces:

1. Patent: EP 0222413, Novel forskolin derivatives.
2. Tochiro Tatee, et al. (1996) Forskolin Derivatives. I. Synthesis, and Cardiovascular and Adenylate Cyclase-Stimulating Activities of Water-Soluble Forskolins. Chem. Pharm. Bull. 44, 2274-2279.
3. Shunichi Kametani et al. (1995) The Pharmacodynamics of 6-(3-Dimethylaminopropionyl)forskolin and a Possible Metabolite in Beagles. J of Pharmaceutical Sciences 85, 377-380.
4. Mutsuhito Kirura et al. (2004) Pharmacokinetics and a simulation model of colforsin daropate, new forskolin derivative inotropic vasodilator, in patients undergoing coronary artery bypass grafting. Pharmacological Research 49, 275-281.
5. Y. Khandelwal et al. (1988) Cardiovascular Effect of New Water-Soluble Derivatives of Forskolin. J. Med. Chem. 31, 1872-1879.
6. A. Laurenza et al. (1987) Stimulation of Adenylate Cyclase by Water-Soluble Analogues of Forsklin. Molecular Pharmacology, 32: 133-139.
7. Bansi Lal et al. (1998) Hydroxyacyl Derivatives of Forskolin - their Positive Inotropic Activity. Bioorganic & Medicinal Chemistry 6, 2061-2073.
8. K. B. Seamon, et al. (1983) Structure-Activity Relationship for Activation of Adenylate Cyclase by the Diterpene Forskolin and Its Derivatives. J. Med. Chem. 26, 436-442.
9. Chou, T. C. et al. (1984) Quantitative analysis of dose-effect relationships: the combined effects of multiple drugs or enzyme inhibitors. Adv. Enzyme Regul., 22, 27-55.
10. Yokoyama, Y. et al. (2000) Synergy between angiostatin and endostatin: inhibition of ovarian cancer growth. Cancer Res., 60, 2190-2196.
11. Zhou, J. R. et al. (2004) Combined inhibition of estrogen-depdendent human breast carcinoma by soy and tea bioactive components in mice. Int. J. Cancer, 108, 8-14.

## Claims

1. A pharmaceutical composition for use in treating psoriasis or psoriatic arthritis, comprising:
a forskolin derivative or a pharmaceutically acceptable salt thereof, and
a prostaglandin compound or a glucocorticoid compound,
wherein the forskolin derivative is selected from the group consisting of 6-(4-aminobutyryl)forskolin, 6-[4-(dimethylamino)butyryl]forskolin, 6-[3-aminopropionyl]forskolin, 6-[3-(methylamino)propionyl]forskolin, and 6-[3-(dimethylamino)propionyl]forskolin.

2. The pharmaceutical compostion for use of claim 1, wherein the pharmaceutically acceptable salt of forskolin derivative is hydrochloride salt.

3. The pharmaceutical composition for use of any one of claims 1-2, wherein the pharmaceutical acceptable salt of forskolin derivative is 6-[3-(dimethylamino) propionyl]forskolin hydrochloride or 6-[3-(methylamino)propionyl]forskolin hydrochloride.

4. The pharmaceutical composition for use of any one of claims 1-3, wherein the prostaglandin compound is prostaglandin E2, or a pharmaceutically acceptable salt thereof.

5. The pharmaceutical composition for use of any one of claims 1-3, wherein the glucocorticoid compound is hydrocortisone, or a pharmaceutically acceptable salt thereof.

6. The pharmaceutical composition for use of any one of claims 1-4, wherein the pharmaceutical composition comprises 6-[3-(dimethylamino)propionyl]forskolin hydrochloride and prostaglandin E2.

7. The pharmaceutical composition for use of any one of claims 1-3, 5 wherein the pharmaceutical composition comprises 6-[3-(dimethylamino)propionyl]forskolin hydrochloride and hydrocortisone.

8. A pharmaceutical composition comprising:
(1) a forskolin derivative selected from the group consisting of 6-(4-aminobutyryl)forskolin, 6-[4-(dimethylamino)butyryl]forskolin, 6-[3-aminopropionyl]forskolin, 6-[3-(methylamino)propionyl]forskolin, and 6-[3-(dimethylamino)propionyl]forskolin, or a pharmaceutically acceptable salt thereof; and
(2) a prostaglandin compound or a glucocorticoid compound.

9. The pharmaceutical composition of claim 8, wherein the pharmaceutical composition comprises:
(1) a forskolin derivative selected from the group consisting of 6-(4-aminobutyryl)forskolin, 6-[4-(dimethylamino)butyryl]forskolin, 6-[3-aminopropionyl]forskolin, 6-[3-(methylamino)propionyl]forskolin, and 6-[3-(dimethylamino)propionyl]forskolin, or a pharmaceutically acceptable hydrochloride salt thereof; and
(2) prostaglandin E2 or a pharmaceutically acceptable salt thereof, or hydrocortisone or a pharmaceutically acceptable salt thereof.

10. The pharmaceutical composition of claim 8 or claim 9, wherein the pharmaceutical composition comprises 6-[3-(dimethylamino)propionyl]forskolin hydrochloride and prostaglandin E2.

11. The pharmaceutical composition of claim 8 or claim 9, wherein the pharmaceutical composition comprises 6-[3-(dimethylamino)propionyl]forskolin hydrochloride and hydrocortisone.

12. The pharmaceutical composition for use of any one of claims 1 to 7 or the pharmaceutical composition of any one of claims 8-11, wherein the pharmaceutical composition for use or the pharmaceutical composition is in a form of tablet, granule, powder, capsule, injection preparation, suppository, drops, external paste, ointment, medicated oil or spray.

## Patentansprüche

1. Pharmazeutische Zusammensetzung zur Verwendung in der Behandlung von Psoriasis oder Psoriasis-Arthritis, umfassend:
ein Forskolin-Derivat oder ein pharmazeutisch akzeptables Salz davon, und
eine Prostaglandin-Verbindung oder eine Glucocorticoid-Verbindung,
wobei das Forskolin-Derivat ausgewählt ist aus der Gruppe bestehend aus 6-(4-Aminobutyryl)forskolin, 6-[4-(Dimethylamino)butyryl]-forskolin, 6-[3-Aminopropionyl]forskolin, 6-[3-(Methylamino)propionyl]-forskolin und 6-[3-(Dimethylamino)propionyl]forskolin.

2. Pharmazeutische Zusammensetzung zur Verwendung nach Anspruch 1, wobei das pharmazeutisch akzeptable Salz des Forskolin-Derivats ein Hydrochlorid-Salz ist.

3. Pharmazeutische Zusammensetzung zur Verwendung nach einem der Ansprüche 1 bis 2, wobei das pharmazeutisch akzeptable Salz des Forskolin-Derivats 6-[3-(Dimethylamino)propionyl]forskolin-Hydrochlorid oder 6-[3-(Methylamino)propionyl]forskolin-Hydrochlorid ist.

4. Pharmazeutische Zusammensetzung zur Verwendung nach einem der Ansprüche 1 bis 3, wobei die Prostaglandin-Verbindung Prostaglandin E2 oder ein pharmazeutisch akzeptables Salz davon ist.

5. Pharmazeutische Zusammensetzung zur Verwendung nach einem der Ansprüche 1 bis 3, wobei die Glucocorticoid-Verbindung Hydrocortison oder ein pharmazeutisch akzeptables Salz davon ist.

6. Pharmazeutische Zusammensetzung zur Verwendung nach einem der Ansprüche 1 bis 4, wobei die pharmazeutische Zusammensetzung 6-[3-(Dimethylamino)propionyl]forskolin-Hydrochlorid und Prostaglandin E2 umfasst.

7. Pharmazeutische Zusammensetzung zur Verwendung nach einem der Ansprüche 1 bis 3 oder 5, wobei die pharmazeutische Zusammensetzung 6-[3-(Dimethylamino)propionyl]forskolin-Hydrochlorid und Hydrocortison umfasst.

8. Pharmazeutische Zusammensetzung, umfassend:
(1) ein Forskolin-Derivat, das aus der Gruppe bestehend aus 6-(4-Aminobutyryl)forskolin, 6-[4-(Dimethylamino)butyryl]forskolin, 6-[3-Aminopropionyl]forskolin, 6-[3-(Methylamino)propionyl]forskolin und 6-[3-(Dimethylamino)propionyl]forskolin ausgewählt ist, oder ein pharmazeutisch akzeptables Salz davon;
und
(2) eine Prostaglandin-Verbindung oder eine Glucocorticoid-Verbindung.

9. Pharmazeutische Zusammensetzung nach Anspruch 8, wobei die pharmazeutische Zusammensetzung umfasst:
(1) ein Forskolin-Derivat, das aus der Gruppe bestehend aus 6-(4-Aminobutyryl)forskolin, 6-[4-(Dimethylamino)butyryl]forskolin, 6-[3-Aminopropionyl]forskolin, 6-[3-(Methylamino)propionyl]forskolin und 6-[3-(Dimethylamino)propionyl]forskolin ausgewählt ist, oder ein pharmazeutisch akzeptables Hydrochlorid-Salz davon; und
(2) Prostaglandin E2 oder ein pharmazeutisch akzeptables Salz davon, oder Hydrocortison oder ein pharmazeutisch akzeptables Salz davon.

10. Pharmazeutische Zusammensetzung nach Anspruch 8 oder 9, wobei die pharmazeutische Zusammensetzung 6-[3-(Dimethylamino)propionyl]-forskolin-Hydrochlorid und Prostaglandin E2 umfasst.

11. Pharmazeutische Zusammensetzung nach Anspruch 8 oder 9, wobei die pharmazeutische Zusammensetzung 6-[3-(Dimethylamino)propionyl]-forskolin-Hydrochlorid und Hydrocortison umfasst.

12. Pharmazeutische Zusammensetzung zur Verwendung nach einem der Ansprüche 1 bis 7 oder pharmazeutische Zusammensetzung nach einem der Ansprüche 8 bis 11, wobei die pharmazeutische Zusammensetzung zur Verwendung oder die pharmazeutische Zusammensetzung in Form von Tabletten, Granulat, Pulver, Kapseln, Injektionszubereitung, Zäpfchen, Tropfen, äußerlicher Paste, Salbe, medikamentenhaltigem Öl oder Spray ist.

## Revendications

1. Composition pharmaceutique pour une utilisation dans le traitement du psoriasis ou de polyarthrite psoriasique, comprenant :
un dérivé de forskoline ou un sel pharmaceutiquement acceptable de celui-ci, et
un composé de prostaglandine ou un composé de glucocorticoïde,
dans lequel le dérivé de forskoline est sélectionné dans le groupe constitué de 6-(4-aminobutyryl)-forskoline, 6-[4-(diméthylamino)-butyryl]-forskoline, 6-[3-aminopropionyl]-forskoline, 6-[3-(méthylamino)-propionyl]-forskoline, et de 6-[3-(diméthylamino)-propionyl]-forskoline.

2. Composition pharmaceutique pour une utilisation selon la revendication 1, dans laquelle le sel pharmaceutiquement acceptable du dérivé de forskoline est un sel chlorhydrate.

3. Composition pharmaceutique pour une utilisation selon l'une quelconque des revendications 1 à 2, dans laquelle le sel pharmaceutiquement acceptable du dérivé de forskoline est un chlorhydrate de 6-[3-(diméthylamino)-propionyl]-forskoline ou un chlorhydrate de 6-[3-(méthylamino)-propionyl]-forskoline.

4. Composition pharmaceutique pour une utilisation selon l'une quelconque des revendications 1 à 3, dans laquelle le composé de prostaglandine est une prostaglandine E2 ou un sel pharmaceutiquement acceptable de celle-ci.

5. Composition pharmaceutique pour une utilisation selon l'une quelconque des revendications 1 à 3, dans laquelle le composé de glucocorticoïde est une hydrocortisone, ou un sel pharmaceutiquement acceptable de celle-ci.

6. Composition pharmaceutique pour une utilisation selon l'une quelconque des revendications 1 à 4, dans laquelle la composition pharmaceutique comprend un chlorhydrate de 6-[3-(diméthylamino)-propionyl]-forskoline et une prostaglandine E2.

7. Composition pharmaceutique pour une utilisation selon l'une quelconque des revendications 1 à 3 et 5, dans laquelle la composition pharmaceutique comprend un chlorhydrate de 6-[3-(diméthylamino)-propionyl]-forskoline et une hydrocortisone.

8. Composition pharmaceutique comprenant :
(1) un dérivé de forskoline sélectionné dans le groupe constitué de 6-(4-aminobutyryl)-forskoline, 6-[4-(diméthylamino)-butyryl]-forskoline, 6-[3-aminopropionyl]-forskoline, 6-[3-(méthylamino)-propionyl]-forskoline, et 6-[3-(diméthylamino)-propionyl]-forskoline, ou un sel pharmaceutiquement acceptable de ceux-ci ; et
(2) un composé de prostaglandine ou un composé de glucocorticoïde.

9. Composition pharmaceutique selon la revendication 8, dans laquelle la composition pharmaceutique comprend :
(1) un dérivé de forskoline sélectionné dans le groupe constitué de 6-(4-aminobutyryl)-forskoline, 6-[4-(diméthylamino)-butyryl]-forskoline, 6-[3-aminopropionyl]-forskoline, 6-[3-(méthylamino)-propionyl]-forskoline, et 6-[3-(diméthylamino)-propionyl]-forskoline, ou un sel chlorhydrate pharmaceutiquement acceptable de ceux-ci ; et
(2) une prostaglandine E2 ou un sel pharmaceutiquement acceptable de celle-ci, ou une hydrocortisone ou un sel pharmaceutiquement acceptable de celle-ci.

10. Composition pharmaceutique selon la revendication 8 ou la revendication 9, dans laquelle la composition pharmaceutique comprend un chlorhydrate de 6-[3-(diméthylamino)-propionyl]-forskoline et une prostaglandine E2.

11. Composition pharmaceutique selon la revendication 8 ou la revendication 9, dans laquelle la composition pharmaceutique comprend un chlorhydrate de 6-[3-(diméthylamino)-propionyl]-forskoline et une hydrocortisone.

12. Composition pharmaceutique pour une utilisation selon l'une quelconque des revendications 1 à 7 ou composition pharmaceutique selon l'une quelconque des revendications 8 à 11, dans laquelle la composition pharmaceutique pour une utilisation ou la composition pharmaceutique est sous forme de comprimés, de granules, de poudre, de gélules, de préparation pour injection, de suppositoires, de gouttes, de crème externe, de pommade, d'huile ou de spray médicamenteux.
